# EUROPEAN PATENT APPLICATION

(11) **EP 2 233 586 A2**
(43) Date of publication of application: **29.09.2010**
(21) Application number: 08865077.5
(22) Date of filing: 18.12.2008
(51) Int. Cl.: C12Q 1/68

(54) **GENETIC MARKERS FOR THE PROGNOSIS OF MULTIPLE SCLEROSIS**

(30) Priority: 21.12.2007 ES 200703415
(71) Applicant: Instituto Cientifico Y Tecnologico De Navarra, S.A, 31008 Pamplona (ES); Progenika Biopharma, S.A., 48160 Derio-Vizcaya (ES)
(72) Inventor: PALACIOS URTASUN, Ricardo, E-31008 Pamplona - Navarra (ES); SEPULCRE BERNAD, Jorge, E-31008 Pamplona - Navarra (ES); VILLOSLADA DÍAZ, Pablo, E-31008 Pamplona - Navarra (ES); MARTÍNEZ MARTÍNEZ, Antonio, E-48160 Derio - Vizcaya (ES); SIMÓN BUELA, Laureano, E-48160 Derio - Vizcaya (ES)
(74) Representative: ABG Patentes, S.L.
(86) International application number: PCT/ES2008/000785
(87) International publication number: WO 2009/080849

(57) **Abstract**

The present invention relates to a series of genes the expression of which is altered in subjects suffering multiple sclerosis with respect to healthy subjects or in subjects suffering multiple sclerosis with a good prognosis with respect to subjects suffering multiple sclerosis with a bad prognosis. A subset formed by 13 genes and two clinical variables which allows predicting the progress of a patient with a high reliability has been validated from an initial set of genes which showed said differential expression. From said expression values, the invention provides methods for predicting the progress of a patient diagnosed with multiple sclerosis from tables of conditional probability between the expression levels of a determined gene or group of genes and the probability that the patient has a good or bad prognosis of the disease.

## Description

### Technical Field of the Invention

The invention relates to a method for the prognosis of multiple sclerosis and, more specifically, to a method for predicting the clinical progress of patients diagnosed with multiple sclerosis by means of analyzing the expression levels of a series of genes.

### Background of the Invention

Multiple sclerosis (MS) has a prevalence of around 70 cases every 100.000 inhabitants in Spain and in Western civilization it is the most common cause of chronic neurological disability in young adults after traffic accidents. Approximately 70% of cases starts between 20 and 40 years of age, with a peak in the age of onset around 25 or 30 years, so the huge impact it has on the professional, family and social life of those affected, as well as the enormous economic and social expense it generates, which is similar to that of Alzheimer's disease, is easily understandable.

Multiple sclerosis is a heterogeneous disease in its presentation and progress in which 80-85% of the patients present a clinical course which progresses to auto-limited flare-ups which, as they are repeated, cause a functional residual deficit (relapsing-remitting form; RR-MS). After 10-15 years of progress, 50% of them will pass to a secondary progressive course of increment of the disability not related to the flare-ups (secondary progressive form; SP-MS), and after 25 years the percentage reaches 90% of the patients. In 10% of the cases the course is progressive from the onset (primary progressive form; PP-MS). 10 to 20% of the patients will remain without significant sequelae 15 years after the onset of the disease (benign forms) and in 1-3% of the cases, however, the patients will progress accumulating a great disability in a few months after the start of the disease (aggressive or fulminant forms).

Interferon beta (Betaferon®, Rebif®, Avonex®) and glatiramer acetate or copolymer (Copaxone®) are the first medicines that have shown beneficial effects in the relapsing-remitting form of multiple sclerosis. These medicines reduce the formation of plaques and the number of flare-ups by one third compared with patients without treatment. Treatment with Natalizumab (Tysabri®) has recently been included in the therapy of multiple sclerosis, having greater effectiveness than prior treatments (they prevent flare-ups by approximately 60%), although with potential serious side effects. However, the individual response to treatment is unpredictable and ranges from excellent to complete ineffectiveness. The lack of biological tests which predict the activity and aggressiveness of the disease prevents prescribing the best treatment to each patient and forces administering a preventive treatment for life with the subsequent economic cost and the effect on the quality of life. Being able to have a predictive test of the aggressiveness and activity of the disease would allow a customized treatment.

Standard methods of diagnosis of multiple sclerosis include determining levels of IgG in CSF, brain imaging by means of magnetic resonance and spinal cord imaging and the exclusion of other autoimmune diseases by means of serum determinations. Although the usefulness of said tests in predicting the course of multiple sclerosis has recently been studied, their predictive capacity is very limited and in clinical practice they are used for diagnostic purposes but not for prognostic purposes or for deciding on or monitoring therapy. Therefore, reaching a diagnosis and a suitable prognosis continues to be a problem.

Different studies of the natural history of multiple sclerosis have allowed identifying some clinical variables associated with the progress of multiple sclerosis. The factors which best predict a relatively benign course are belonging to the female sex, the onset of the disease at an early age (less than 30 years), uncommon attacks, a relapsing-remitting pattern and a mild nature of the disease in the studies by means of magnetic resonance of the central nervous system. In contrast, the factors which predict a more aggressive course are the male sex and late onset (because it is associated with the progressive forms), the recurrence of the second flare-up in the first year after the first flare-up, accumulating disability early on, the clinical onset with motor or coordination symptoms and the persistence of sequela after the first flare-up, and especially reaching certain disability levels (Kurtzke Expanded Disability Status Scale EDSS: 3.0, 4.0, 6.0) at early ages.

Until now, the attempt has been made to develop methods of diagnosis and prognosis based on the detection of autoantibodies in serum (Bielekova, B. and, Martin R. Brain. 2004 Jul;127(Pt 7):1463-78.; Berger, T. and Reindl, M., 2006, Disease Markers, 22:207-212). For example, Berger, T. et al. (New England J. Medicine, 2003, 146:181-197) have described that the presence of anti-myelin antibodies (anti-MOG) are capable of predicting the risk of first relapse in patients suffering a clinically isolated syndrome, suggesting multiple sclerosis. However, subsequent well-designed studies have not been able to confirm their predictive usefulness (Kuhle J. et al., N Engl J Med. 2007, 356:371-8.; Pelayo R. et al. N. Engl. J. Med. 2007, 356:426-8.)

However, until now no antibody has been identified which meets the requirements of a diagnostic or prognostic biomarker of multiple sclerosis. Nor is the simultaneous determination of several autoantibodies of use, and it furthermore involves great difficulties and a high cost.

In addition, gene expression studies (transcriptome) by means of DNA chips allow having a global vision of the genes that are participating in a process, so this type of analysis could become a valuable clinical tool for the diagnosis or prognosis of MS.

Until now differences have been described in the expression levels of various genes in multiple sclerosis, which could be candidates as biomarkers of multiple sclerosis (reviewed in Goertsches, R. et al., 2006, Current Pharmaceutical Design, 12:3761-3779). These studies compared the expression patterns between patients with multiple sclerosis and controls, but the association between said patterns and the progressive course and prognosis of the disease were never specifically studied.

Ramanathan et al (J. Neuroimmunol., 2001, 116:213-219) have described 34 genes differentially expressed in RR-MS patients in comparison with healthy subjects, most of them related to inflammatory and immune processes.

Bomprezzi et al. (Human Molecular Genetics, 2003, 12:2191-2199) identified a series of genes the expression levels of which in PBMCs allows distinguishing RR-MS and SP-MS patients and healthy volunteers. By means of this approach, over a thousand genes which allowed distinguishing samples of multiple sclerosis from controls could be identified. The strongly dominant genes included HSP70 and the CDC28 protein kinase (CKS) 2 which, combined with histone H1 of the (HIF) 2 family and the PAFAH1B1, respectively, allowed a good discrimination between multiple sclerosis and controls. These pairs had a prediction value of 80% for classifying an independent sample in the right class. A correlation between the most discriminatory pairs of genes and relevant biological pathways of multiple sclerosis was also observed. Such molecules, which were highly expressed in multiple sclerosis included CD27, TNF receptor, the alpha locus of the T cell receptor and its associated chain ZAP70, and the zinc finger protein (ZNF) 148. Furthermore, the IL-7 receptor (IL-7R), which is required for the development of T and B cells, was also strongly overexpressed. The repressed genes in multiple sclerosis were HSP70 and CKS2, both involved in apoptosis regulation. It has previously been suggested that HSP70 can be an autoantigen in multiple sclerosis, but it can also be involved in the degradation of mRNA in the ubiquitin-proteasome pathway. The activation of the remodeling process of the extracellular matrix was evident due to the overexpression of the matrix metalloproteinase (MMP) - 19 and the repression of a TIMP 1 inhibitor.

The expression patterns for multiple sclerosis and the pathophysiology of the disease have been analyzed in several studies. Iglesias et al. (J. Neuroimmunol., 2004 150:163-77) identified a system of 553 genes differentially expressed in RR-MS compared with the healthy controls, 87 of which were highly significant. Among the genes differentially expressed, some involved in the activation and co-stimulation of T cells could be identified, which included several interferon response genes, such as IL-12, CD40, cytotoxic antigen 4 (CTLA4), T cell receptors, immunoglobulins, the IL-6 receptor, the IL-8 receptor, and integrins, for example VLA4 and VLA6, as well as different genes of the E2F pathway (E2F2, E2F3, CDC25A, CDK2), the thymopoietin (TMPO), and PRIM1. The importance of the E2F pathway in multiple sclerosis was validated in experimental autoimmune encephalitis (EAE). E2F1-deficient mice showed only a mild course of the EAE disease.

The gene patterns for the activity of multiple sclerosis have also been studied. International patent application WO03081201 identified a pattern of 1109 genes in PBMCs from 26 multiple sclerosis patients compared with healthy volunteers regardless of the state of the activation of the disease. The pattern was validated with the LOOCV method, which gave only two errors in the classification, proving that the patterns observed represent a true biological phenomenon. These genes included those related to activation and expansion, inflammatory stimuli of the T cell (cytokines and integrins), spreading epitope, and apoptosis. Comparison of the profiles of the expression in PBMCs of multiple sclerosis patients in a flare-up showed a pattern of 721 genes. Protease L, CTSL1 and the MCP1 and MCP2 proteins were overexpressed during the flare-up. In contrast, several genes related to apoptosis such as cyclin G1 and the caspases (CASP) 2, 8 and 10 were repressed.

W003023056 describes methods for the diagnosis of and/or the susceptibility to multiple sclerosis by means of determining variations in the expression levels of 25 genes.

Individually, a gene (CX3CR1) which has been identified in expression analysis in sub-populations of T cells has been proposed as a marker for the activity of the disease. CX3CR1 is repressed in RR-MS and PP-MS patients compared with healthy volunteers. This finding has been validated by real-time PCR and by flow cytometry in independent cohorts. The NK cells are responsible for the phenotype, whereas the expression of CX3CR1 is not altered in CD8 cytotoxic cells in multiple sclerosis patients with respect to healthy controls.

US2004/0091915 describes a method for predicting the survival rate of patients diagnosed with multiple sclerosis by means of detecting a deletion in the CCR5 gene.

W02005054810 describes a method for predicting the survival rate of patients diagnosed with multiple sclerosis by means of detecting a deletion in the gene CD24.

In W003001212 describes a method for the diagnosis of multiple sclerosis based on detecting in a sample isolated from the subject the absence of the wt-SARG-1 protein or of the mRNA which encodes it.

US20050064483 describes a method for monitoring the response of a multiple sclerosis patient to treatment with interferon-beta or with glatiramer acetate by means of detecting variations in the expression of at least 4 genes selected from a group of 34 genes.

US20050089919 describes a method for detecting multiple sclerosis which comprises detecting variations in the expression of at least one gene selected from a series of 31 genes.

US20050164253 describes a method for detecting multiple sclerosis which comprises detecting variations in the expression of at least one gene selected from the group of RIPK2, NFKBIE, TNFAIP3, DAXX, TNFSF10, BAG1, TOP1, ADPRT, CREB1, MYC, BAG4, RBBP4, GZMA, BCL2 and E2F5.

US20060115826 describes a method for the diagnosis of multiple sclerosis which comprises detecting variations in the expression of at least two genes selected from a set of 107 genes associated with inflammatory processes.

WO0207921 describes a method for the diagnosis of multiple sclerosis which comprises detecting variations in the expression of a selected gene panel in that they show variations in their expression level in an experimental animal model of autoimmune encephalitis. In this study, the analysis of the expression of the different genes was conducted by means of a human DNA chip in which about 14000 different genes were represented.

WO03081201 describes a method for the diagnosis of multiple sclerosis based on detecting variations in the expression of a gene panel represented in a human DNA chip which contained 12625 human genes.

W003095618 describes methods for the diagnosis of multiple sclerosis, for the differential diagnosis of multiple sclerosis with respect to lateral amyotrophic sclerosis, for predicting the response of a subject diagnosed with multiple sclerosis to a treatment with Avonex by means of detecting variations of the expression of a series of genes involved in different signaling pathways.

However, all the methods described until now have been aimed at detecting differences between patients suspected of presenting multiple sclerosis and control subjects, whereby they have an essentially diagnostic use, but they do not allow predicting the progress of the disease in patients who have already been diagnosed with multiple sclerosis. Therefore, there is a need for methods which allow predicting the progress of the disease in patients already diagnosed.

### Summary of the Invention

In a first aspect, the invention relates to an *in vitro* method for determining the clinical prognosis of a patient who has multiple sclerosis which comprises
(a) comparing
   (i) the value corresponding to the expression of a gene selected from the group of KLHDCS, CASP2, EMID1, PRO1073 BTBD7, MGC2518, WDR20bis, NEK4, SYLT2. DOCK10, TTC10, PTPRC and CTLA4 with a table of conditional probabilities between ranges of modal values of the expression of said genes and probability values that the multiple sclerosis has a good or bad prognosis and/or
   (ii) the value of a clinical variable selected from the group of EDSS and MSFC with a table of conditional probabilities between ranges of modal values of said clinical variables and probability values that the multiple sclerosis has a good or bad prognosis and
(b) assigning a probability of a bad and a good prognosis corresponding to the probability associated with the range in which the value of the expression or of the clinical variable is located.

In another aspect, the invention relates to an *in vitro* method for determining the clinical prognosis of a patient who has sclerosis which comprises
(a) comparing
   (i) the values corresponding to the expression of at least two genes selected from the group of KLHDC5, CASP2, EMID1, PRO1073, BTBD7, MGC2518, WDR20bis, NEK4, SYLT2. DOCK10, TTC10, PTPRC and CTLA4 with a table of conditional probabilities between ranges of modal values of the expression of said genes and probability values that the multiple sclerosis has a good or bad prognosis and/or
   (ii) the values of the EDSS and MSFC clinical variables with a table of conditional probabilities between ranges of modal values of said clinical variables and probability values that the multiple sclerosis has a good or bad prognosis and
(b) assigning a probability of a bad prognosis corresponding to the conditional probability of a bad prognosis associated with the ranges of modal values in which the expression values of each of the genes the expression of which has been determined and/or the clinical variables determined are located and assigning a probability of a good prognosis corresponding to the conditional probability of a good prognosis associated with the ranges of modal values in which the expression values for each of the genes the expression of which has been determined and/or the clinical variables determined are located

In another aspect, the invention relates to a method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from the group of genes listed in positions 3, 5, 6, 7, 9, 11, 13, 16, 19, 20, 22, 24, 25, 26, 30, 31, 33, 34, 35, 37, 41 or 43 of Table 3, or of the polypeptides encoded by said genes, in a biological sample isolated from the patient and
(b) comparing the expression levels of said genes or of said polypeptides with a reference value calculated from one or several samples obtained from a healthy patient,
wherein
(i) an increase of the expression of the genes in position 6, 7, 9, 33, 35, 37 or 43, or of the polypeptides encoded by said genes, or a reduction of the expression of the genes in position 3, 5, 11, 13, 16, 19, 22, 24, 25, 26, 30, 31, 34, 41, or of the polypeptides encoded by said genes with respect to the reference value, is indicative of a bad prognosis of multiple sclerosis in said subject, that the therapy is ineffective or that the patient is selected for an aggressive therapy or
(ii) an increase of the expression of the genes in positions 3, 5, 11, 16, 20, 30, or of the polypeptides encoded by said genes, or a reduction of the expression of the gene in position 43, or of the polypeptide encoded by said gene with respect to the reference value, is indicative of a good prognosis of multiple sclerosis in said patient, that the therapy is effective or that the patient is selected to not receive therapy or to receive a rather non-aggressive therapy.

In another aspect, the invention relates to a method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from the group of genes listed in positions 1 to 21 of Table 5, or of the polypeptides encoded by said genes, in a biological sample isolated from the patient and
(b) comparing the expression levels of said genes or of said polypeptides with a reference value calculated from one or several samples obtained from patients with a good prognosis and with a reference value calculated from one or several samples obtained from patients with a bad prognosis
wherein
(i) an increase of the expression of the genes in position 1, 2, 3, 4, 5, 8, 9, 10, 14, 19, 20 or 21 or of the polypeptides encoded by said genes with respect to a reference value obtained from one or several samples from patients diagnosed with multiple sclerosis with a bad prognosis is indicative of a good prognosis of multiple sclerosis in said subject, that the therapy is effective or that the patient is selected to not receive an aggressive therapy and
(ii) an increase of the expression of the genes in positions 6, 7, 11, 12, 13, 15, 16, 17 or 18 or of the polypeptides encoded by said genes with respect to a reference value obtained from one or several samples from patients diagnosed with multiple sclerosis with a good prognosis is indicative of a bad prognosis of multiple sclerosis in said patient, that the therapy is not effective or that the patient is selected to receive therapy or to receive a rather non-aggressive therapy.

In another aspect, the invention relates to a method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from Table 6, or of the polypeptides encoded by said genes, in a sample isolated from the patient and
(b) comparing the expression levels of said genes with a reference value calculated from one or several samples obtained from a healthy patient
wherein an increase of the expression of the genes in position 4, 8, 11, 13, 15, 18, 19, 20, 21, 24, 25, 28, 30 or 32, or of the polypeptides encoded by said genes, or a reduction of the genes in position 1, 2, 3, 5, 6, 7, 9, 10, 12, 14, 16, 17, 22, 23, 26, 27, 29 or 31, or of the polypeptides encoded by said genes, with respect to the reference value is indicative of a bad prognosis of multiple sclerosis, that the therapy is not effective or that the patient is selected for an aggressive therapy.

In another aspect, the invention relates to a method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from Table 7, or of the polypeptides encoded by said genes, in a sample isolated from the patient and
(b) comparing the expression levels of said genes with a reference value calculated from one or several samples obtained from a healthy patient
wherein an increase of the expression of the genes in position 2, 5, 6, 7, 8 and 10, or of the polypeptides encoded by said genes, or a reduction of the expression of the genes in position 1, 3, 4 or 9, or of the polypeptides encoded by said genes, with respect to the reference value is indicative of a good prognosis of multiple sclerosis or that the therapy administered is effective or that the patient is selected to not receive therapy or to receive a rather non-aggressive therapy.

In another aspect, the invention relates to a method for diagnosing multiple sclerosis in a subject which comprises
(a) determining the expression level of one or several genes selected from the group of genes indicated in Table 8, or of the polypeptides encoded by said genes, in a sample isolated from the subject and
(b) comparing the expression levels of said genes with a reference value calculated from one or several samples obtained from a healthy patient
wherein a reduction of the expression of the genes in position 1, 2, 6, 10, 15 or 16, or of the polypeptides encoded by said genes, or an increase in the expression of the genes in position 3, 4, 5, 7, 8, 9, 11, 12, 13 or 14, or of the polypeptides encoded by said genes, with respect to the reference value is indicative that the subject suffers multiple sclerosis.

In another aspect, the invention relates to a kit comprising a set of probes wherein said set comprises a probe specific for each of the genes indicated in at least one table selected from the group of Tables 3, 5-8 and 11.

In another aspect, the invention relates to the use of a kit of the invention for determining the prognosis of a patient diagnosed with multiple sclerosis, for determining the effectiveness of a treatment for multiple sclerosis or for diagnosing multiple sclerosis in a patient.

### Brief Description of the Drawings

Figure 1: GO distribution of the 45 genes which presented significant differences between the three classes.
Figure 2: Cluster analysis of the samples.
Figure 3: Cluster analysis of the genes.

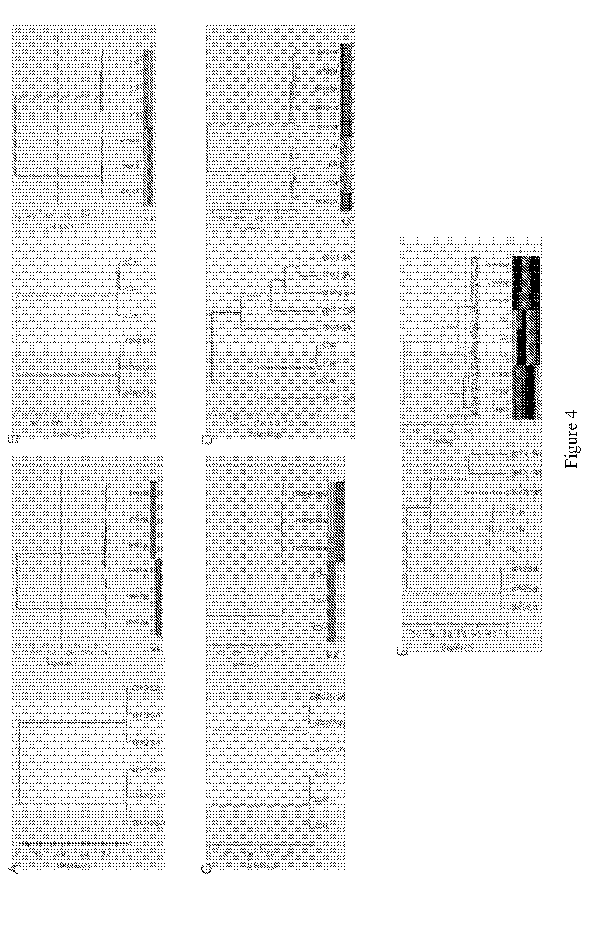

Figure 4: Cluster analysis of both the samples and the genes obtained after the comparisons with p<0.001 of the classes (A) good and bad prognosis, (B) control and bad prognosis, (C) control and good prognosis, (D) control and multiple sclerosis and the comparison with p<0.005 of the three classes (E).
Figure 5: Graphic representation in the 60 studied samples of the behavior of the 25 genes which presented significant differences (p<0.01) between the three classes.
Figure 6: Bayesian network and confusion matrix of the validation of the classifier using the EDSS (Kurtzke Expanded Disability Status Scale) and MSFC (Multiple Sclerosis Functional Composite) clinical variables and those genes which presented significant differences (p<0.01) in the expression levels of the three classes.
Figure 7: Graphic representation in the 40 samples from patients of the behavior of the 13 genes which presented significant differences (p<0.05) between good and bad prognosis.
Figure 8: Bayesian network and confusion matrix of the validation of the classifier using the clinical variables (EDSS and MSFC) and those genes which presented significant differences (p<0.05) in the expression levels between good and bad prognosis.

### Detailed Description of the Invention

The authors of the present invention, using DNA microarrays, have identified a series of genes which are differentially expressed in patients diagnosed with multiple sclerosis in which the disease has a good prognosis with respect to patients in which the disease shows a bad prognosis or to control subjects. Likewise, the authors of the invention have identified a series of genes the expression of which is modified in patients diagnosed with multiple sclerosis in which the disease has a bad prognosis. From an initial set of identified genes, a subset of 13 genes was validated by means of real-time PCR the expression variations of which allowed predicting the type of prognosis of the patients in a significant manner (p<0.05).

Thus, in a first aspect, the invention relates to an *in vitro* method (hereinafter the first method of the invention) for determining the clinical prognosis of a patient who has multiple sclerosis which comprises
(a) comparing
   (i) the value corresponding to the expression of a gene selected from the group of KLHDC5, CASP2, EMID1 PRO1073, BTBD7, MGC2518, WDR20bis, NEK4, SYLT2, DOCK10, TTC10, PTPRC and CTLA4 with a table of conditional probabilities between ranges of modal values of the expression of said genes and probability values that the multiple sclerosis has a good or bad prognosis and/or
   (ii) the value of a clinical variable selected from the group of EDSS and MSFC with a table of conditional probabilities between ranges of modal values of said clinical variables and probability values that the multiple sclerosis has a good or bad prognosis and
(b) assigning a probability of a bad and a good prognosis corresponding to the probability associated with the range in which the value of the expression or of the clinical variable is located.

Additionally, from the expression levels of these 13 genes and by using two clinical variables (ESSS and MSFC), a classifier was obtained which allowed predicting the progress of the disease with a precision of 95%. Thus, in another aspect, the invention relates to an *in vitro* method (hereinafter the first method of the invention) for determining the clinical prognosis of a patient who has sclerosis which comprises
(a) comparing
   (i) the values corresponding to the expression of at least two genes selected from the group of KLHDC5, CASP2, EMID1, PRO1073, BTBD7, MGC2518, WDR20bis, NEK4, SYLT2. DOCK10, TTC10, PTPRC and CTLA4 with a table of conditional probabilities between ranges of modal values of the expression of said genes and probability values that the multiple sclerosis has a good or bad prognosis and/or
   (ii) the values of the EDSS and MSFC clinical variables with a table of conditional probabilities between ranges of modal values of said clinical variables and probability values that the multiple sclerosis has a good or bad prognosis and
(b) assigning a probability of a bad prognosis corresponding to the conditional probability of a bad prognosis associated with the ranges of modal values in which the expression values of each of the genes the expression of which has been determined and/or the clinical variables determined are located and assigning a probability of a good prognosis corresponding to the conditional probability of a good prognosis associated with the ranges of modal values in which the expression values for each of the genes the expression of which has been determined and/or the clinical variables determined are located.

According to the present invention, "determining the clinical prognosis" is understood as giving an opinion as to the future condition of the patient (clinical (physical and cognitive) disability) after a determined number of years (e.g. 2, 5, 10 years from the moment of the opinion). The clinical prognosis can be performed in recently diagnosed patients or after the first flare-up, as well as at any time of the course of his disease. The condition of the patient can be defined based on the symptoms of multiple sclerosis, including a reduced capacity for controlling small movements, reduced attention span, reduced coordination, reduced discerning capacity, reduced memory, depression, difficulty in speaking or understanding language, dizziness, double vision, eye discomfort, facial pain, fatigue, loss of balance, problems with movement that are slowly progressive and begin in the legs, muscle atrophy, muscle spasms (especially in the legs), muscle spasticity (uncontrollable spasm of muscle groups), numbing or abnormal sensation in any area, pain in the arms and legs, paralysis of one or both arms or legs, bad pronunciation, tingling sensation, shaking in one or both arms or legs, uncontrollable and rapid eye movements, increased urinary frequency, difficulty in urinating, urinary urgency, urinary incontinence, vertigo, loss of vision, walk/gait anomalies and weakness in one or both arms or legs.

"Modal value" is understood in the context of the present invention as a value of the variable (in this case, of the expression levels) which partitions the range of values of said variable into two or more sub-ranges. Suitable methods for determining said value have been described in Dougherty, J. et al., (Proc. of the 12th International Conference on Machine Learning; 1995. p. 194-202) and by Liu H. et al. (Data Mining and Knowledge Discovery, 2002, 6:393-423), the content of which is incorporated in the present application in its entirety. In a preferred embodiment, in order to obtain said modal value the variable is discretized by means of a supervised learning algorithm (computational) of the more informative discretization thresholds with respect to a reference variable (in the present invention, the diagnosis). To calculate the discretization ranges, if starting from the ordered sequence of values Ai= {v1, v2, ..., vn}, the information gain is evaluated with respect to a reference variable for all the possible partitions (n - 1). The partition with the greatest information gain is the one that is used for comparing with the remaining attributes. The decisions of the nodes will be [A[x] < vi] and [A[x] ≥ vi].

Possible discretization algorithms suitable for their use in the present invention include the decision tree, the equal frequency algorithm and the equal distance algorithm. In a preferred embodiment, the discretization algorithm is the decision tree.

"Tables of conditional probabilities" is understood in the context of the present invention as a table in which the possible modal values of the expression of a determined gene or clinical variable are represented, and in which each of the modal values is correlated with a determined probability that the disease of the patient will follow a positive or negative prognosis. In a preferred embodiment, the tables of conditional probabilities between the modal values of the expression of each of the genes and the probability values that the multiple sclerosis has a good or bad prognosis and between the modal values of each of the clinical variables and the probability values that the multiple sclerosis has a good or bad prognosis are those indicated in Table 14.

"KLHDC5 gene" is understood as the gene encoding the kelch domain containing 5 protein the human variant of which is described in the GenEMBL database under accession number BC108669.

"CASP2 gene" is understood as the gene encoding the precursor of caspase 2. The human form of said gene is described in the GenEMBL database under accession numbers U13021 and U13022.

"EMID1 gene" is understood as the gene encoding the precursor of the EMI domain-containing protein 1. The human form of said gene is described in the GenEMBL database under accession number AJ416090.

"PRO1073 gene" is understood as the gene described in the GenEMBL database under accession number AF113016.

"BTBD7 gene" is understood as the gene encoding the BTB/POZ domain-containing protein 7. The human form of said gene is described in the GenEMBL database under accession number BX538231.

"MGC25181 1 gene" is understood as the gene encoding the hypothetical MGC25181 protein. The human form of said gene is described in the GenEMBL database under accession number AC114730.

"WDR20bis gene" is understood as the gene encoding the WD repeat-containing protein 20. The human form of said gene is described in the GenEMBL database under accession number BC028387.

"NEK4 gene" is understood as the gene encoding the serine/threonine kinase Nek4. The human form of said gene is described in the GenEMBL database under accession number L20321.

"SYLT2 gene" is understood as the gene encoding the Synaptotagmin-like protein 2. The human form of said gene is described in the GenEMBL database under accession number AK000170.

"DOCK10 gene" is understood as the gene encoding the dedicator of cytokinesis protein 10. The human form of said gene is described in the GenEMBL database under accession number AB014594.

"TTC10 gene" is understood as the gene encoding the tetratricopeptide repeat protein 10. The human form of said gene is described in the GenEMBL database under accession number U20362.

"PTPRC gene" is understood as the gene encoding the protein tyrosine phosphatase receptor type C. The human form of said gene is described in the GenEMBL database under accession number BC01786

"CTLA4 gene" is understood as the gene encoding the precursor of cytotoxic T-lymphocyte protein 4. The human form of said gene is described in the GenEMBL database under accession number AF414120.

"EDSS" is understood as the Kurtzke Expanded Disability Status Scale, as it is defined in Kurtzke, J.F. (Neurology, 1983, 33:1444-1452).

"MSFC" is understood as the Multiple Sclerosis Functional Composite, as is defined in Fischer, J. S. et al. (National MS Society Clinical Prognoses Assessment Task Force. Mult. Scler. 1999, 5:244-250).

The determination of the expression values of a nucleic acid is performed by means of the relative measurement of the expression levels of a gene of interest compared to the expression levels of a reference nucleic acid. Said measurements can be carried out by any method known by the person skilled in the art, such as those included in Sambrook, J. et al. (Molecular Cloning: A Laboratory Manual. 2nd ed., Cold Spring Harbor Laboratory, Cold Spring Harbor Laboratory Press, Cold Spring Harbor, NY (1989)) and Ausubel et al. (Current Protocols in Molecular Biology, eds. Ausubel et al, John Wiley & Sons (1992)). Typical processes for detecting the polynucleotide resulting from the transcription of a gene of interest include the extraction of RNA from a cell or tissue sample, hybridization of said sample with a labeled probe, i.e., with a nucleic acid fragment having a sequence complementary to the molecule of nucleic acid to be detected, and detection of the probe (for example, by means of Northern blotting). The invention also contemplates the detection of the expression levels of a determined gene by means of using primers in a polymerase chain reaction (PCR), such as anchor PCR, RACE PCR, ligase chain reaction (LCR). In a preferred embodiment, the determination of the modal values of the expression is carried out by means of real-time PCR.

These methods include the steps of collecting a cell sample from a subject, isolating the mRNA from said samples, converting the mRNA present in the sample into complementary DNA (cDNA), contacting the cDNA preparation with one or several primers which specifically hybridize with the target gene in suitable conditions for the hybridization and amplification of said nucleic acid followed by the detection of the presence of an amplification product. Alternative amplification methods include self-sustained sequence replication (Guatelli, JC. et al., (1990) Proc. Natl. Acad. Sci. USA 87:1874-1878), transcriptional amplification system (Kwoh, D.E. et al., (1989) Proc. Natl. Acad. Sci. USA 86:1173-1177), Q-Beta replicase (Lizardi, P.M. et al. (1988) BioTechnology 6:1197) or any other known nucleic acid amplification method, followed by the detection of the amplified molecules using techniques that are well known by the person skilled in the art. These methods of detection are particularly suitable for detecting nucleic acids when said molecules are present in a very reduced number of copies.

In other embodiments, the genes *per se* can be used as markers of multiple sclerosis. For example, the increase of the expression of a determined gene can be due to the duplication of the corresponding gene, such that the duplication can be used as a diagnosis of the disease. The detection of the number of copies of a target gene can be carried out using methods that are well known by the person skilled in the art. The determination of the number of copies of a determined gene is typically carried out by means of Southern blot in which the complete DNA of a cell or of a tissue sample is extracted, hybridized with a labeled probe and said probe is detected. The labeling of the probe can be by means of a fluorescent compound, by means of an enzyme or an enzymatic cofactor. Other typical methods for the detection/quantification of DNA include direct sequencing, column chromatography and quantitative PCR using standard protocols.

The determination of the expression levels of a gene can be carried out in any biological sample from a subject, including different types of biological fluids, such as blood, serum, plasma, cerebrospinal fluid, peritoneal fluid, feces, urine and saliva, as well as tissue samples. The biological fluid samples can be obtained by any conventional method as can the tissue samples; by way of illustration said tissue samples can be biopsy samples obtained by surgical resection.

The second method of the invention contemplates the simultaneous determination of the expression values of a larger number of genes. Thus, the second method of the invention can include the determination of the expression values of at least 3, at least 4, at least 5, at least 6, at least 7, at least 8, at least 9, at least 10 and at least 11 genes.

In a preferred embodiment, the second method of the invention requires the determination of the expression values of the KLHDC5 gene and of the EDSS clinical variable. In another preferred embodiment, the second method of the invention requires additionally determining the expression value of the CASP2 gene. In another preferred embodiment, the expression value of the EMID1 gene is additionally determined. In an even more preferred embodiment, the value of the MSFC clinical variable is additionally determined. In an even more preferred embodiment, the method additionally comprises determining the expression value of the PRO1073 gene. In another preferred embodiment, the second method of the invention includes additionally determining the expression value of the BTBD7 gene. In an even more preferred embodiment, the method involves additionally determining the expression value of the MGC2518 gene. In another embodiment, the method of the invention involves additionally determining the expression value of the WDR20bis gene. In an even more preferred embodiment, the method of the invention involves additionally determining the expression value of the NEK4 gene. In another embodiment, the method of the invention involves additionally determining the expression value of the DOCK10 gene. In another preferred embodiment, the method of the invention involves additionally determining the expression value of the TTC10 gene. In an even more preferred embodiment, the method of the invention involves additionally determining the expression value of the PTPRC gene. In another preferred embodiment, the method of the invention involves additionally determining the expression value of the CTLA4 gene.

The inventors have additionally shown the existence of different genes which are differentially expressed in patients diagnosed with multiple sclerosis with a bad prognosis with respect to patients diagnosed with multiple sclerosis with a good prognosis and with respect to control subjects, which allows the development of prognostic methods for predicting the development of the disease. The authors of the present invention have additionally shown the existence of genes which are differentially expressed in subjects diagnosed with multiple sclerosis with respect to healthy patients, which allows the use of said genes for diagnostic purposes.

Thus, in another aspect, the invention relates to a method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from the group of genes listed in positions 3, 5, 6, 7, 9, 11, 13, 16, 19, 20, 22, 24, 25, 26, 30, 31, 33, 34, 35, 37, 41 or 43 of Table 3, or of the polypeptides encoded by said genes, in a biological sample isolated from the patient and
(b) comparing the expression levels of said genes or of said polypeptides with a reference value
wherein
(i) an increase of the expression of the genes in position 6, 7, 9, 33, 35, 37 or 43 or of the polypeptides encoded by said genes or a reduction of the expression of the genes in position 3, 5, 11, 13, 16, 19, 22, 24, 25, 26, 30, 31, 34, 41 or of the polypeptides encoded by said genes is indicative of a bad prognosis of multiple sclerosis in said subject, that the therapy is ineffective or that the patient is selected for an aggressive therapy or
(ii) an increase of the expression of the genes in positions 3, 5, 11, 16, 20, 30 or of the polypeptides encoded by said genes or a reduction of the expression of the gene in position 43 or of the polypeptide encoded by said gene is indicative of a good prognosis of multiple sclerosis in said patient, that the therapy is effective or that the patient is selected to not receive therapy or to receive a rather non-aggressive therapy.

In another aspect, the invention relates to a method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from the group of genes listed in positions 1 to 21 of Table 5, or of the polypeptides encoded by said genes, in a biological sample isolated from the patient and
(b) comparing the expression levels of said genes or of said polypeptides with a reference value calculated from one or several samples obtained from patients with a good prognosis and with a reference value calculated from one or several samples obtained from patients with a bad prognosis
wherein
(i) an increase of the expression of the genes in position 1, 2, 3, 4, 5, 8, 9, 10, 14, 19, 20 or 21 or of the polypeptides encoded by said genes with respect to a reference value obtained from one or several samples from patients diagnosed with multiple sclerosis with a bad prognosis is indicative of a good prognosis of multiple sclerosis in said subject, that the therapy is effective or that the patient is selected to not receive an aggressive therapy and
(ii) an increase of the expression of the genes in positions 6, 7, 11, 12, 13, 15, 16, 17 or 18 or of the polypeptides encoded by said genes with respect to a reference value obtained from one or several samples from patients diagnosed with multiple sclerosis with a good prognosis is indicative of a bad prognosis of multiple sclerosis in said patient, that the therapy is not effective or that the patient is selected to receive therapy or to receive a rather non-aggressive therapy.

In another aspect, the invention relates to a method for identifying the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from Table 6 in a sample isolated from the patient and
(b) comparing the expression levels of said genes with a reference value
   wherein an increase of the expression of the genes in position 4, 8, 11, 13, 15, 18, 19, 20, 21, 24, 25, 28, 30 or 32 or a reduction of the genes in position 1, 2, 3, 5, 6, 7, 9, 10, 12, 14, 16, 17, 22, 23, 26, 27, 29 or 31 with respect to the reference value is indicative of a bad prognosis of multiple sclerosis, that the therapy is not effective or that the patient is selected for an aggressive therapy.

In another aspect, the invention relates to a method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from Table 7 in a sample isolated from the patient
(b) comparing the expression levels of said genes with a reference value
   wherein an increase of the expression of the genes in position 2, 5, 6, 7, 8 and 10 or a reduction of the expression of the genes in position 1, 3, 4 or 9 with respect to the reference value is indicative of a good prognosis of multiple sclerosis or that the therapy administered is effective or that the patient is selected to not receive therapy or to receive a rather non-aggressive therapy.

In another aspect, the invention relates to a method for diagnosing multiple sclerosis in a subject which comprises
(a) determining the expression level of one or several genes selected from the group of genes indicated in Table 8 in a sample isolated from the subject
(b) comparing the expression levels of said genes with a reference value
   wherein a reduction of the expression of the genes in position 1, 2, 6, 10, 15 or 16 or an increase in the expression of the genes in position 3, 4, 5, 7, 8, 9, 11, 12, 13 or 14 with respect to the sample control is indicative that the subject suffers multiple sclerosis.

The definition of "determination of the clinical prognosis" has been described above.

"Monitoring the effect of the therapy administered to a subject who has multiple sclerosis" is understood according to the present invention as determining if a therapy has any incidence on the prognosis.

"Assigning a customized therapy to a subject who has multiple sclerosis" is understood as deciding, based on the prognosis of an individual, on the most suitable type of therapy for preventing the occurrence of the previously indicated symptoms. In cases of worse prognosis, a more aggressive therapy is applied from the time that said worse prognosis is detected. Thus, more aggressive therapies include immune modulators to aid in controlling the immune system, including interferons (Avonex, Betaseron or Rebif), monoclonal antibodies (Tysabri) and glatiramer acetate (Copaxone) and chemotherapy.

"Reference value" is understood as a measurement of the expression of a determined gene or polypeptide that can be calculated or established from one or several control samples. These can come from a healthy subject, from a subject with multiple sclerosis, or from subjects with a good or a bad prognosis, according to the objective of the method.

The person skilled in the art will understand that the determination of the expression levels of the genes included in Tables 3, 5, 6, 7 and 8 can be carried out using techniques known by the person skilled in the art.

The determination of the expression levels of a nucleic acid relating to the levels of a reference nucleic acid can be carried out by any method known by the person skilled in the art, as has been described above.

In other embodiments, the genes *per se* can be used as markers of multiple sclerosis in those cases in which the increase of the expression of a determined gene can be due to the duplication of the corresponding gene, such that the duplication can be used as a diagnosis of the disease. The detection of the number of copies of a target gene can be carried out using the methods described above.

Alternatively, the invention contemplates methods for determining the clinical prognosis of a subject who has multiple sclerosis or for monitoring the effect of the therapy administered to a subject who has multiple sclerosis or for assigning a therapy to a subject who has multiple sclerosis in which the expression level of one or several proteins encoded by the genes which are indicated in Tables 1 to 4 is determined. In this aspect, the invention requires the extraction of a protein sample from a cell or tissue sample followed by the incubation of said sample with a labeled reagent capable of binding specifically to said sample (for example, an antibody) and detecting said reagent, wherein the marker which includes the reagent is selected from the group of a radioisotope, a fluorescent compound, an enzyme or an enzymatic cofactor.

Typical immunodetection methods include ELISA, RIA, immunoradiometric assay, fluoroimmunoassay, chemoluminescent assays, bioluminescent assays and Western blot assays.

Generally all the immunoassays include a step in which a sample suspected of containing a determined antigen or in which the concentration of said antigen is to be known is contacted with a first antibody in suitable conditions for the formation of the immune complexes. Suitable samples for the determination include a tissue section or biopsy, a tissue extract or a biological fluid. Once the antigen-antibody complexes have been formed, the preparation is subjected to one or several washings to remove antibodies that have not specifically bound.

Then, the detection of the immune complexes is performed by means of methods that are well known by the person skilled in the art, such as radioactive, fluorescent, or biological methods or methods based on the determination of an enzymatic activity.

For the purpose of increasing sensitivity, it is possible to use an additional ligand, such as a second antibody or a ligand coupled to biotin, for example. In this situation, an additional incubation step for incubating the ligand-antibody complexes obtained in the first step with the second antibody in suitable conditions for the formation of the secondary immune complexes is necessary. The secondary complexes are subjected to a washing cycle to remove secondary antibodies which have non-specifically bound, and then the amount of secondary immune complex is determined by means of determining the signal emitted by the secondary antibody.

Additional methods include the detection of the primary immune complexes by means of a two-step process. In this process, a secondary ligand (an antibody), which has binding affinity for the antibody forming part of the immune complexes, is contacted with said complexes to form secondary immune complexes, as was described above. After a washing step, the secondary immune complexes are contacted with a tertiary ligand or antibody which binds with high affinity to the secondary antibody to give rise to the formation of the tertiary complexes. The third ligand or antibody is bound to a detectable marker which allows the detection of the tertiary immune complexes.

Other detection methods include Western blotting, dot blotting, FACS analysis and the like. In one embodiment, the antibodies directed against the antigens of the invention are immobilized on a surface showing affinity for the proteins (for example polystyrene). Then a composition in which the antigen to be detected is present is added. After a washing step to remove the non-specifically bound complexes, the bound antigen can be detected by means of a second antibody which is coupled to a detectable marker. This type of ELISA is referred to as sandwich ELISA. The detection can also be carried out by means of adding a second antibody and a third antibody having affinity for the second antibody and which is bound to a detectable marker.

In another type of ELISA, the samples containing the antigen are immobilized and are detected by means of a competitive method in which the sample in which the antigen to be detected is present is mixed with antibodies labeled for said antigen and is added on the surface in which the antibody is immobilized. The presence of antigen in the sample prevents the binding of the antibody to the immobilized antigen such that the amount of antibody that binds to the immobilized antigen is present in an inverse proportion with respect to the amount of antigen in the sample to be analyzed.

It is also possible to detect the antigen by means of immunohistochemistry and confocal microscopy in tissue sections obtained from frozen samples, fixed in formaldehyde or embedded in paraffin using techniques that are widely known by the person skilled in the art.

The reference sample which is used for determining the variation of the expression levels of the genes and proteins used in the present invention. In one embodiment, the reference value is obtained from the signal provided using a tissue sample obtained from a healthy individual. Samples are preferably taken from the same tissue of several healthy individuals and are pooled, such that the amount of mRNA or of polypeptides in the sample reflects the mean value of said molecules in the population.

The method of the present invention can be combined with other diagnostic methods (e.g. oligoclonal bands in the CSF, neuroimaging (MR, OCT), clinical variables (disability scales, rate of flare-ups, age, sex) or biological markers: a) genetic markers (polymorphisms, haplotypes); b) pathological patterns in biopsy; c) antibodies, etc.

The methods of the present invention are particularly useful for establishing the prognosis in patients who have suffered a single flare-up of multiple sclerosis, in a patient suffering RR-MS or in a patient suffering PP-MS. This method would therefore be applied once during task of diagnosing the disease. It could also be applied to patients with the disease already diagnosed but in which, given the great variability of the disease, it is unknown if the disease is stable or if it will progress, again with a prognostic nature and to decide on the treatment. Therefore, the vast majority of patients would take the test at least once, except those with the disease in a very advanced stage in which the bad progress is already obvious and in which there are not possibilities of choosing between treatments. A sub-group of patients could take the test on several occasions if, over the years, the clinical course of the disease seems to change and the prognosis is to be re-assured.
- In the case of having a favorable prognosis, the physician may recommend periodic follow-up and assess if any immunomodulating treatment is still required, being able to choose the most convenient or comfortable for the patient given the mild nature of his disease. This information is also critical for the patient because he can make important decisions about his life, such as getting married, having children, the type of work, the stress level and risks in his life, medical insurance, life insurance, type of home, etc.
- In the event that the prognosis is unfavorable, the physician would more strongly recommend immunomodulating treatments and probably use from the start the most effective second line treatments (for example, Tysabri) or administer combined therapy or chemotherapy. In addition, the patient can express in a more informed manner the risks he can undertake due to the more aggressive therapy that is considered, as well as decide about his life in vital aspects such as getting married, having children, the type of job, the stress level and risks in his life, medical insurance, life insurance, type of home, etc.

In principle, any sample isolated from a patient can be used in the methods of the invention. Thus, the determination of the mRNA or polypeptide levels can be performed in a tissue biopsy or in a biological fluid (serum, saliva, semen, sputum, CSF, tears, mucous, sweat, milk and the like). The determination can be carried out in tissue homogenates or in more or less clarified fractions thereof. In a preferred embodiment, the determination of the mRNA and polypeptide levels of the invention is carried out from mononuclear cell extracts obtained from peripheral blood.

In the event that the expression levels of several of the genes identified in the present invention are to be determined simultaneously, compositions containing at least one copy of a probe specific for each of the genes indicated in at least one table selected from the group of Tables 3, 5-8 and 11 are useful.

Thus, in another aspect, the invention relates to a kit comprising a probe specific for each of the genes indicated in at least one table selected from the group of Tables 3, 5-8 and 11.

"Kit" is understood in the context of the present invention as a product containing the different reagents necessary for carrying out the methods of the invention packaged so as to allow their transport and storage. Materials suitable for packaging the components of the kit include glass, plastic (polyethylene, polypropylene, polycarbonate and the like), bottles, vials, paper, sachets and the like. Additionally, the kits of the invention can contain instructions for the simultaneous, sequential or separate use of the different components in the kit. Said instructions can be in the form of printed material or in the form of an electronic medium capable of storing instructions such that they can be read by a subject, such as electronic storage media (magnetic discs, tapes and the like), optical media (CD-ROM, DVD) and the like. The media can additional or alternatively contain Internet addresses which provide said instructions.

In a preferred embodiment, the kit of the invention consists of a probe specific for each of the genes indicated in at least one table selected from the group of Tables 3, 5-8 and 11.

In a preferred embodiment, the genes forming part of the array are the genes indicated in Table 11 and at least one reference gene.

In another preferred embodiment, the probes or the antibodies forming the kit of the invention are coupled to an array.

In the event that the expression levels of several of the genes identified in the present invention are to be determined simultaneously, the inclusion of probes for all the genes the expression of which is to be determined in a hybridization microarray is useful.

The microarrays comprise a plurality of nucleic acids spatially distributed and stably associated with a support (for example, a biochip). The nucleic acids have a sequence complementary to particular subsequences of the genes the expression of which is to be detected, so they are capable of hybridizing with said nucleic acids. In the methods of the invention, a microarray comprising an array of nucleic acids is contacted with a nucleic acid preparation isolated from the patient object of study. The incubation of the microarray with the nucleic acid preparation is carried out in suitable conditions for hybridization. Subsequently, after the elimination of the nucleic acids that have not been retained in the support, the hybridization pattern is detected, which provides information about the genetic profile of the analyzed sample. Although the microarrays are capable of providing both qualitative and quantitative information of the nucleic acids present in a sample, the invention requires the use of arrays and methodologies capable of providing quantitative information.

The invention contemplates a variety of arrays in terms of type of probes and in terms of type of support used. The probes included in the arrays which are capable of hybridizing with the nucleic acids can be nucleic acids or analogs thereof which maintain the hybridization capacity, such as, for example, nucleic acids in which the phosphodiester bond has been replaced with a phosphorothioate, methylimino, methylphosphonate, forforamidate, guanidine bond and the like, nucleic acids in which the ribose of the nucleotides has been replaced with another hexose, peptide nucleic acids (PNA). The length of the probes can be 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and they can be single-stranded or double-stranded nucleic acids.

The selection of the probes specific for the different target genes is carried out such that they bind specifically to the target nucleic acid with minimum hybridization to unrelated genes. However, there are probes 20 nucleotides in length which are not unique for a determined mRNA. Therefore, probes directed against said sequences will show cross-hybridization with identical sequences appearing in mRNA of unrelated genes. In addition, there are probes which do not specifically hybridize with the target genes in the conditions used (due to secondary structures or interactions with the substrate of the array). Probes of this type should not be included in the array. Therefore, the person skilled in the art will note that the probes that are going to be incorporated in a determined array must be optimized before their incorporation in the array. The optimization of the probes is generally carried out by generating an array containing a plurality of probes directed against the different regions of a determined target polynucleotide. This array is contacted first of all with a sample containing the target nucleic acid in an isolated manner and, second of all, with a complex mixture of nucleic acids. Probes showing a highly specific hybridization with the target nucleic acid but not a low or any hybridization with the complex sample are thus selected for their incorporation in the arrays of the invention. It is additionally possible to include in the array hybridization controls for each of the probes that is going to be studied. In a preferred embodiment, the hybridization controls contain an altered position in the central region of the probe. In the event that high levels of hybridization are observed between the studied probe and its hybridization control, the probe is not included in the array.

In a preferred embodiment, the array contains a plurality of probes complementary to subsequences of the target nucleic acid of a constant length or of a variable length in a range of 5 to 50 nucleotides. The array can contain all the specific probes of a determined mRNA of a determined length or it can contain probes selected from different regions of an mRNA. Each probe is assayed in parallel with a probe with a changed base, preferably in the central position of the probe. The array is contacted with a sample containing nucleic acids with sequences complementary to the probes of the array and the hybridization signal is determined with each of the probes and with the corresponding hybridization controls. Those probes in which a greater difference between the hybridization signal with the probe and its hybridization control is observed are selected. The optimization process can include a second optimization round in which the hybridization array is hybridized with a sample which does not contain sequences complementary to the probes of the array. After the second round of selection, those probes presenting hybridization signals less than a threshold level will be selected. Probes which exceed both controls, i.e., that show a minimum level of non-specific hybridization and a maximum level of specific hybridization with the target nucleic acid, are thus selected.

The microarrays of the invention contain not only probes specific for the polynucleotides indicative of a determined pathophysiological situation, but they also contain a series of control probes, which can be of three types: normalization controls, expression level controls and hybridization controls.

Normalization controls are oligonucleotides which are perfectly complementary to labeled reference sequences which are added to the nucleic acid preparation to be analyzed. The signals derived from the normalization controls after hybridization provide an indication of the variations in the hybridization conditions, intensity of the label, efficiency of the detection and another series of factors that can result in a variation of the hybridization signal between different microarrays. The signals detected from the remaining probes of the array are preferably divided by the signal emitted by the control probes thus normalizing the measurements. Virtually any probe can be used as a normalization control. However, it is known that the effectiveness of the hybridization ranges according to the nucleotide composition and the length of the probe. Therefore, preferred normalization probes are those which represent the mean length of the probes present in the array, although they can be selected such that they comprise a range of lengths which reflect the remaining probes present in the array. The normalization probes can be designed such that they reflect the mean nucleotide composition of the remaining probes present in the array. A limited number of normalization probes is preferably used, selected such that they suitably hybridize, i.e., they do not present a secondary structure and they do not show sequence similarity with any of the probes of the array. The normalization probes can be located in any position in the array or in multiple positions in the array to efficiently control variations in the hybridization efficiency related to the structure of the array. The normalization controls are preferably located in the corners of the array and/or in the center thereof.

The expression level controls are probes which specifically hybridize with genes which are constitutively expressed in the sample which is analyzed. The expression level controls are designed to control the physiological condition and the metabolic activity of the cell. The analysis of the covariance of the expression level control with the expression level of the target nucleic acid indicates if the variations in the expression levels are due to changes in the expression levels or if they are due to changes in the global transcription rate in the cell or in its general metabolic activity. Thus, in the case of cells presenting deficiencies in a determined metabolite essential for cell viability, it is expected that a reduction in both the expression levels of the target gene and in the expression levels of the control will be observed. In addition, if an increase in the expression of the target gene and of the control gene is observed, it is probable that it is due to an increase of the metabolic activity of the cell and not to a differential increase in the expression of the target gene. Any probe which corresponds to a constitutively expressed gene can be used, such as genes encoding proteins which perform essential functions of the cells, such as β-2-microglobulin, ubiquitin, ribosomal 18S protein, cyclophilin A, transferrin receptor, actin, GAPDH and the like. In a preferred embodiment, the expression level controls are GAPDH, tyrosine 3-monooxygenase/triptophan 5-monooxygenase activation protein (YWHAZ), ubiquitin, beta-actin and β-2-microglobulin.

The hybridization controls can be included for the probes directed against the target genes and for the probes directed against the expression level or against the normalization controls. Error controls are oligonucleotide probes identical to the probes directed against the target genes but they contain mutations in one or several nucleotides, i.e., they contain nucleotides in certain positions which do not hybridize with the corresponding nucleotide in the target gene. The hybridization controls are selected such that, by applying the suitable hybridization conditions, the target gene should hybridize with the specific probe but not with the hybridization control, or with a reduced efficiency. The hybridization controls preferably contain one or several modified positions in the center of the probe. The hybridization controls therefore provide an indication of the degree of non-specific hybridization or of cross-hybridization to a nucleic acid in the sample to a probe different from the one containing the exactly complementary sequence.

The arrays of the invention can also contain amplification and sample preparation controls which are probes complementary to subsequences of control genes selected because they typically do not appear in the biological sample object of study, such as probes for bacterial genes. The RNA sample is supplemented with a known amount of a nucleic acid which hybridizes with the selected control probe. The determination of the hybridization to said probe indicates the degree of recovery of the nucleic acids during its preparation as well as an estimate of the alteration caused in the nucleic acids during the processing of the sample.

Once a set of probes which show the suitable specificity and a set of control probes are available, the latter are arranged in the array in a known position such that, after the hybridization and detection steps, it is possible to establish a correlation between a positive hybridization signal and the particular gene from the coordinates of the array in which the positive hybridization signal is detected.

The microarrays can be high density arrays with thousands of oligonucleotides by means of *in situ* photolithographic synthesis methods (Fodor et al., 1991, Science, 767-773). Probes of this type are typically redundant, i.e., they include several probes for each mRNA to be detected.

In a preferred embodiment, the arrays are low density arrays, or LDA, containing less than 10000 probes in each one per square centimeter. In said low density arrays, the different probes are manually applied with the aid of a pipette in different locations of a solid support (for example, a glass surface, a membrane). The supports used for fixing the probes can be obtained from a wide variety of materials, including plastic, ceramic, metals, gels, membranes, glass and the like. The microarrays can be obtained using any methodology known by the person skilled in the art.

After hybridization, in the cases in which the non-hybridized nucleic acid is capable of emitting a signal in the detection step, a washing step is necessary to remove said non-hybridized nucleic acid. The washing step is carried out using methods and solutions known by the person skilled in the art.

In the event that the labeling in the nucleic acid is not directly detectable, it is possible to connect the microarray comprising the target nucleic acids bound to the array with the other components of the system necessary for producing the reaction which gives rise to a detectable signal. For example, if the target nucleic acids are labeled with biotin, the array is contacted with streptavidin conjugated with a fluorescent reagent in suitable conditions so that the binding occurs between the biotin and the streptavidin. After the incubation of the microarray with the system which generates the detectable signal, it is necessary to perform a washing step to remove all the molecules which have non-specifically bound to the array. The washing conditions will be determined by the person skilled in the art using suitable conditions according to the system which generates the detectable signal which has been used and which are well known by the person skilled in the art.

The resulting hybridization pattern can be viewed or detected in different ways, said detection being determined by the type of system used in the microarray. Thus, the detection of the hybridization pattern can be carried out by means of scintillation counting, autoradiography, determination of a fluorescent signal, calorimetric determinations, detection of a light signal and the like.

Before the detection step, it is possible to treat the microarrays with an endonuclease specific for single-stranded DNA, such that the DNA which has non-specifically bound to the array is removed, whereas the double-stranded DNA resulting from the hybridization of the probes of the array with the nucleic acids of the sample object of study remains unchanged. Endonucleases suitable for this treatment include S 1 nuclease, Mung bean nuclease and the like. In the event that the treatment with endonuclease is carried out in an assay in which the target nucleic acid is not labeled with a directly detectable molecule (for example, in an assay in which the target nucleic acid is biotinylated), the treatment with endonuclease will be performed before contacting the microarray with the other members of the system which produces the detectable signal.

After hybridization and the possible subsequent washing and treatment processes, the hybridization pattern is detected and quantified, for which the signal corresponding to each hybridization point in the array is compared to a reference value corresponding to the signal emitted by a known number of terminal-labeled nucleic acids to thus obtain an absolute value of the number of copies of each nucleic acid that is hybridized at a determined point of the microarray.

In the event that the expression levels of several of the proteins identified in the present invention are to be determined simultaneously, compositions containing at least one antibody specific for each of the genes indicated in at least one table selected from the group of Tables 1 to 4 are useful. The antibody arrays such as those described by De Wildt et al. (2000) Nat. Biotechnol. 18:989-994; Lueking et al. (1999) Anal. Biochem. 270:103-111; Ge et al. (2000) Nucleic Acids Res. 28, e3, 1-VII; MacBeath and Schreiber (2000) Science 289:1760-1763; WO 01/40803 and WO 99/51773A1, are useful for this purpose. The antibodies of the array include any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies, or DABS, Fv, scFv and the like. The techniques for preparing said antibodies are well known by the person skilled in the art and include the methods described by Ausubel et al. (Current Protocols in Molecular Biology, eds. Ausubel et al., John Wiley & Sons (1992)).

The antibodies of the array can be applied at high speed, for example, using commercially available robotic systems (for example, those produced by Genetic Microsystems or Biorobotics). The substrate of the array can be nitrocellulose, plastic, glass or it can be made from a porous material such as, for example, archylamide, agarose or another polymer. In another embodiment, it is possible to use cells which produce the antibodies specific for detecting the proteins of the invention by means of their culture in array filters. After the inducing the expression of the antibodies, the latter are immobilized in the filter in the position of the array where the producer cell is arranged.

An antibody array can be contacted with a labeled target and the level of binding of the target to the immobilized antibodies can be determined. If the target is not labeled, a sandwich-type assay can be used which uses a second labeled antibody specific for the polypeptide which binds to the polypeptide which is immobilized in the support.

The quantification of the amount of polypeptide present in the sample in each point of the array can be stored in a database as an expression profile. The antibody array can be produced in duplicate and used for comparing the binding profiles of two different samples

In another aspect, the invention relates to the use of a kit of the invention for determining the prognosis of a patient diagnosed with multiple sclerosis, for determining the effectiveness of a treatment for multiple sclerosis or for diagnosing multiple sclerosis in a patient.

The invention is described below by means of the following examples which must be considered as illustrative and non-limiting of the scope of the invention.

### EXAMPLES

### MATERIALS AND METHODS

### 1. Screening with DNA chips

6 multiple sclerosis patients were recruited, 3 of them were diagnosed as having a bad prognosis and 3 as having a good prognosis, and 3 healthy controls without any history of any autoimmune disease. The prognosis of the patients was determined by means of clinical data associated with the progress of multiple sclerosis in studies of the natural history of multiple sclerosis as a first flare-up type, time until the second flare-up, number of flare-ups in the first 2 to 5 years and initial sequelae (Table 1).

**Table 1: Clinical markers of good and bad prognosis**

| **Clinical prognostic markers** | **Assessment** **+ good prognosis** **- bad prognosis** | **Literature reference** |
|---|---|---|
| Clinical signs of onset related to cerebellum, pyramidal tract or brainstem. | - | 1,2,5,9,6,13,17. |
| Clinical signs of onset related to altered senses or optic neuritis. | + | 1,2,9,13 |
| Polysymptomatic clinical signs of onset (involvement of three or more functional systems) | - | 1,8,7 |
| Time to 2^{nd} flare-up < 1 year. | - | 1,2,4,9,13 |
| Two or more flare-ups in the first two years. | + | 1,2,4,5,13,17. |
| Time greater than or equal to 5 years to EDSS of 3 | + | 1,4,13,17. |
| Persistence of the initial clinical signs for more than 1 year. | - | 1,9,8 |
| Good recovery after the first two flare-ups: (assessment a year after the flare-up with EDSS less than or equal to 1.5). | + | 2,7, |

### Literature references.-

1.- M.A. Hernández Pérez. Factores pronósticos en la EM. Neurología 2001; 16 [supl 1]: 37-42.
2.- Scott TF, Schramke CJ, Novero J, Chieffe C. Short-term prognosis in early relapsing-remitting multiple sclerosis. Neurology 2000 Sep 12;55(5): 689-93.
3.- Brex PA, Ciccarelli O, O'Riordan JI, Sailer M, Thompson AJ, Miller DH. A longitudinal study of abnormalities on MRI and disability from multiple sclerosis. N Engl J Med 2002 Jan 17; 346(3): 158-64.
4.- Weinshenker BG, Bass B, Rice GP, Noseworthy J, Carriere W, Baskerville J, Ebers GC. The natural history of multiple sclerosis: a geographically based study. 2. Predictive value of the early clinical course. Brain 1989 Dec;112 ( Pt 6):1419-28.
5.- Weinshenker BG, Rice GP, Noseworthy JH, Carriere W, Baskerville J, Ebers GC.The natural history of multiple sclerosis: a geographically based study. 3. Multivariate analysis of predictive factors and models of outcome. Brain 1991 Apr;114 ( Pt 2):1045-56.
6.- Miller DH, Hornabrook RW, Purdie G.J. The natural history of multiple sclerosis: a regional study with some longitudinal data. Neurol Neurosurg Psychiatry 1992 May;55(5):341-6.
7.- Runmarker B, Andersen O. Prognostic factors in a multiple sclerosis incidence cohort with twenty-five years of follow-up. Brain 1993 Feb;116 (Pt 1):117-34.
8.- Runmarker B, Andersson C, Oden A, Andersen O. Prediction of outcome in multiple sclerosis based on multivariate models. J Neurol 1994 Oct;241(10):597-604.
9.- Phadke JG. Clinical aspects of multiple sclerosis in north-east Scotland with particular reference to its course and prognosis. Brain 1990 Dec;113 ( Pt 6):1597-628.
10.- Avasarala JR, Cross AH, Trotter JL. Oligoclonal band number as a marker for prognosis in multiple sclerosis. Arch Neurol 2001 Dec;58(12):2044-5.
11.- Lin X, Blumhardt LD. Inflammation and atrophy in multiple sclerosis: MRI associations with disease course. J Neurol Sci 2001 Aug 15;189(1-2):99-104
12.- Simon JH. Brain and spinal cord atrophy in multiple sclerosis. Neuroimaging Clin N Am 2000 Nov;10(4):753-70,ix.
13.- Multiple sclerosis. McAlpine's. Third edition. Alastair Compston. Churchill Livingstone.
14.- Kappos L, Moeri D, Radue EW, Schoetzau A, Schweikert K, Barkhof F, Miller D, Guttmann CR, Weiner HL, Gasperini C, Filippi M. Predictive value of gadolinium-enhanced magnetic resonance imaging for relapse rate and changes in disability or impairment in multiple sclerosis: a meta-analysis. Gadolinium MRI Meta-analysis Group. Lancet 1999 Mar 20;353(9157):964-9
15.- Rovaris M, Filippi M. Contrast enhancement and the acute lesion in multiple sclerosis. Neuroimaging Clin N Am 2000 Nov;10(4):705-16,viii-ix.
16.- Losseff NA, Miller DH, Kidd D, Thompson AJ. The predictive value of gadolinium enhancement for long term disability in relapsing-remitting multiple sclerosis--preliminary results. Mult Scler 2001 Feb;7(1):23-5.
17.- Esclerosis múltiple, Bases clínicas y patogénicas. Cedric S. Raine, Henry F. McFarland, Wallace W. Tourtellotte. Edimsa.

The purification of total RNA was performed from peripheral blood using the PAXgeneTM Blood RNA Kit of PreAnalytiX. The use of this kit allows preserving the RNA expression profile after performing the blood extraction. During the purification of the total RNA, a treatment with DNase was performed to remove the possible DNA contamination. The samples were concentrated by means of Speed-vac and the quality and amount of the RNA purified was estimated by means of testing an aliquot in agarose gel and spectrophotometric measurement.

cDNA was synthesized from 6 µg of total RNA with the SuperScript Choice System Kit of Life Technologies, following the protocol of the Expression Analysis Technical Manual of Affymetrix. cRNA was synthesized from this cDNA following the protocol of the BioArray HighYield RNA Transcript Labeling Kit (T7) of Enzo. The cRNA thus synthesized was purified with the Clean-up module Kit of Affymetrix, being recovered in a final volume of 22 µl of water. Once synthesized and purified, the cRNA was fragmented (15 µg of each sample) to prepare the hybridization mixtures.

The hybridization and the development and scanning of the chips were performed following the protocols and equipment officially recommended by Affymetrix Inc. The chip used was HG-U133 Plus 2.0. The results of the chip were analyzed using the Microarray Suite 5.0 software (MAS 5.0; Affymetrix®) and Biometric Research Branch (BRB) Array Tools 3.2.3 (Dr. Richard Simon and Amy Peng Lam).

### 2. Validation by real-time PCR and construction of the classifier

40 multiple sclerosis patients were recruited, 20 of them were diagnosed as having a bad prognosis and 20 as having a good prognosis, and 20 healthy controls without any history of any autoimmune disease using the clinical criteria described above.

The purification of total RNA was performed from peripheral blood mononuclear cells. By means of centrifuging with a density gradient using Ficoll-Paque of Pharmacia Biotech, the lymphocytes and monocytes were purified and immediately immersed in an RNAlater RNA Stabilization Reagent of Qiagen to preserve the gene expression patterns. The total RNA was purified using the RNeasy Mini Kit of Qiagen and during purification DNA residue was removed by means of treatment with DNase using the RNase-Free DNase Set of Qiagen. The synthesis of cDNA from total RNA was performed using the High-Capacity cDNA Archive Kit of Applied Biosystems.

The gene validation analysis was performed using the Low Density Arrays (LDAs; Applied Biosystems) technology. The LDAs contain 384 wells. The wells contain TaqMan assays validated by Applied Biosystems and the distribution of the assays is configurable by the user. In this project, the chosen plate design is 95 genes +1 control analyzed in duplicate and with two samples studied in each plate.

Taking into account that for LDAs only those assays which are inventoried by Applied Biosystems can be selected, the process for selecting the most suitable assays from the genes to be validated was according to the following criteria:
- The distance from the probe set of Affymetrix to the probe of Applied Biosystems will be the smallest possible.
- The assay should not detect genomic DNA.
- A minimum of four constitutional genes will be selected for the normalization process.

As a first step of the analysis, those samples presenting a standard deviation between replicas of the same PCR assay greater than 0.38 were ruled out. 0.38 is used as the limit value because it indicates that there is a difference of 0.75 between the minimum and the maximum Ct. Since each Ct is equivalent to a PCR cycle and in each cycle the amount of DNA is duplicated, the standard deviation of 0.38 indicates that there is almost twice the amount of DNA in one well than in the other. Then, and to calculate the expression values of each gene, the formula 2exp(Ctmin -Ctsample) is applied, where Ctmin is the minimum Ct value of each gene in all the samples and Ctsample is the Ct value of that gene in that sample.

By using the expression values of the constitutive genes after the processing (5 in this case; GAPDH, YWHAZ, UBC, ACTB, B2M) the normalization factor of each sample was calculated by means of the geNorm program (http://medgen.ugent.be/~jvdesomp/genorm/), which will calculate the geometric mean of the expression value of a number of constitutive or internal control genes. These internal control genes were chosen according to those genes in which it was observed that there was less variation in their expression between the studied conditions in the gene expression analysis experiment in DNA chips previously performed. Once the normalization factor of each sample was obtained, the data of each gene in each sample was normalized with respect to this normalization factor obtained for said sample of the geNorm program.

For the statistical analysis, the data normalized with respect to the control genes were transformed to logarithmic scale (base 2). For the calculation of significant genes, a non-parametric test was applied, which will depend on if 2 (Mann-Whitney U for 2 independent samples) or 3 conditions (Kruskal Wallis H for 3 independent samples) are compared. In all the cases, the p values<0.05 were considered significant differences whereas the p values<0.01 were considered very significant differences. The statistical analysis was performed using the SPSS 11.0 program (SPSS Inc., Chicago, USA).

The Bayesian classifier was constructed using the Bayesian analysis software BayesiaLab 3.2 (Bayesia SA. Laval Cedex, France). To that end, the variables were previously discretized into a maximum of four ranges using the Decision Tree discretization algorithm taking the variable diagnosis as a reference. The learning was performed using the Augmented Naive Bayes algorithm.

### RESULTS

### 1. Screening with DNA chips

Table 2 shows the demographic and clinical characteristics of the multiple sclerosis patients and of the healthy controls used to perform the screening with DNA chips.

**Table 2: Demographic and clinical characteristics**

| | Healthy controls | Good prognosis | Bad prognosis |
|---|---|---|---|
| N | 3 | 3 | 3 |
| Man/Woman | 1/2 | 1/2 | 1/2 |
| Age (years) | 33.0 ± 2.94 | 38.0 ± 6.83 | 33.8 ± 7.63 |
| EDSS score | | 0.75 ± 0.50 | 2.86 ± 1.18 |
| Duration of disease (years) | | 7.00 ± 0.82 | 1.25 ± 0.50 |
| Flare-ups in the last year | | 0.25 ± 0.50 | 2.50 ± 0.58 |

After normalizing the expression levels using MAS 5.0, the probes (genes) were filtered using the BRB Array Tools 3.2.3 according to the following criteria:
1. Those genes which presented an intensity value of less than 10 were assigned said value.
2. A gene was eliminated if less than 20% of the values of the expression data had at least a change of 1.5 in any direction of the value of the median.
3. A gene was eliminated if the percentage of lost or filtered data exceeded 50%.
4. A gene was eliminated if the percentage of values of the missing expression data exceeded 70%.

After filtering, 4,705 genes of the initial 54,675 complied with the criteria. A class comparison test was performed with these genes and 45 of them which presented significant differences between the three classes (control, bad and good prognosis) with a p value<0.001 (Table 3) were identified.

**Table 3: Genes which presented significant differences between the three classes (control, bad and good prognosis) with a p value<0.001.**

| | **P value** | **Healthy controls** | **Bad prognosis** | **Good prognosis** | **Probe** | **Description** | **Annotation** | **Gene symbol** | **Lists or genes** |
|---|---|---|---|---|---|---|---|---|---|
| **1** | 2e-07 | 15.9 | 39.7 | 26 | 11557278 s at | CDNA FLJ33199 fis, clone ADRGL2006377 | Info | | |
| **2** | 3e-07 | 20.6 | 10 | 10 | 229190 at | CDNA FLJ90295 fis, clone NT2RP2000240. | Info | | |
| **3** | 7.4e-06 | 37.8 | 10.7 | 42.7 | 205306 x at | kynurenine 3-monooxygenase (kynurenine 3-hydroxylase) | Info | KMO | |
| **4** | 1.07e-05 | 59.8 | 36.3 | 35.4 | 227541 at | WD repeat domain 20 | Info | WDR20 | |
| **5** | 1.19e-05 | 36.8 | 10.4 | 45.9 | 235401 s at | Fc receptor homolog expressed in B cells | Info | FREB | |
| **6** | 2.07e-05 | 61 | 135.6 | 62.4 | 223226 x at | single stranded DNA binding protein 4 | Info | SSBP4 | |
| **7** | 2.13e-05 | 10.6 | 22.6 | 10 | 210436 at | chaperonin containing TCP1, subunit 8 (theta) | Info | CCT8 | |
| **8** | 6.14e-05 | 117.5 | 69 .7 | 80.6 | 2249445 at | BTB (POZ) domain containing 7 | Info | BTBD7 | |
| **9** | 9.61e-05 | 10 | 20.3 | 10.7 | 1570043 at | | Info | | |
| **10** | 0.0001169 | 19.6 | 56.4 | 28.8 | 219805 at | hypothetical protein FLJ22965 | Info | FL J22965 | |
| **11** | 0.0002011 | 24.6 | 10.4 | 33.8 | 240394 at | | Info | | |
| **12** | 0.0002318 | 46 | 10 | 25.9 | 232383 at | transcription factor EC | Info | TFEC | |
| **13** | 0.0002505 | 31.6 | 12.9 | 32.9 | 221138 s at | | Info | | |
| **14** | 0.0002614 | 116.8 | 52.7 | 70.8 | 232914 s at | synaptotagmin-like 2 | Info | SYTL2 | |
| **15** | 0.0002713 | 41.4 | 11.8 | 35 | 204634 at | NIMA (never in mitosis gene a)-related kinase 4 | Info | NEK4 | |
| **16** | 0.0002741 | 127.5 | 62.9 | 133.1 | 201302 at | annexin A4 | Info | ANXA4 | |
| **17** | 0.0003003 | 82.2 | 35.7 | 38.2 | 216944 s at | inositol 1,4,5-triphosphate receptor, type 1 | Info | ITPR1 | |
| **18** | 0.0003018 | 40.5 | 10 | 21.2 | 235412 at | | Info | | |
| **19** | 0.0003059 | 66 | 40.2 | 61.5 | 203333 at | kinesin-associated protein 3 | Info | KIFAP3 | |
| **20** | 0.0003135 | 10.8 | 10.5 | 25 | 215151 at | dedicator of cytokinesis 10 | Info | DOCK10 | |
| **21** | 0.0003224 | 96.8 | 30.2 | 81.1 | 233558 s at | FLJ12716 protein | Info | FIJ12716 | |
| **22** | 0.0003732 | 155.2 | 80.9 | 158.1 | 217301 x at | retinoblastoma binding protein 4 | Info | RBBP4 | |
| **23** | 0.0003848 | 20.3 | 43.6 | 16.3 | 208050 s at | caspase 2, apoptosis-related cysteine protease (neural precursor cell expressed, developmentally down-regulated 2) | Info | CASP2 | apoptosis, immunology |
| **24** | 0.0004095 | 40.1 | 17 | 42.7 | 36920 at | myotubularin 1 | Info | MTMI | |
| **25** | 0.0004232 | 26.2 | 10 | 23.7 | 225963 at | KIAA1340 protein | Info | KIAA1340 | |
| **26** | 0.0004529 | 34.5 | 11.6 | 36.8 | 212310 at | C219-reactive | Info | FLJ39207 | |
| **27** | 0.0004554 | 45.3 | 10.5 | 34.3 | 235177 at | similar to hepatocellular carcinoma-associated antigen HCA557b | Info | LOC151194 | |
| **28** | 0.0004832 | 61.7 | 21.2 | 67 | 227268 at | PTD016 protein | Info | LOC51136 | |
| **29** | 0.000489 | 422 | 205.2 | 370.8 | 208612 at | glucose regulated protein 58kDa | Info | GRP58 | |
| **30** | 0.0005587 | 29.2 | 10 | 36.7 | 213659 at | zinc finger protein 75 (D8C6) | Info | ZNF75 | |
| **31** | 0.0005844 | 147.3 | 88.7 | 152.8 | 217980 s at | mitochondrial ribosomal protein L16 | Info | MRPL16 | |
| **32** | 0.0005845 | 68.7 | 21.2 | 45.6 | 205584 at | chromosome X open reading frame 45 | Info | CXorf45 | |
| **33** | 0.0006039 | 11.1 | 49 | 11.9 | 220366 at | epididymal sperm binding protein 1 | Info | ELSPBP1 | |
| **34** | 0.0006295 | 140.1 | 75.7 | 134.7 | 201440 at | DEAD (Asp-Glu- Ala-Asp) box polypeptide 23 | Info | DDX23 | |
| **35** | 0.0006427 | 12 | 24.9 | 10 | 23990 x at | | Info | | |
| **36** | 0.0006527 | 53.3 | 28.5 | 63 | 204703 at | tetratricopeptide repeat domain 10 | Info | TTC10 | |
| **37** | 0.0007041 | 16.7 | 40.1 | 12.1 | 216129 at | ATPase, Class II, type 9A | Info | ATP9A | |
| **38** | 0.0007168 | 42.2 | 25.1 | 56.5 | 218536 at | MRS2-like, magnesium homeostasis factor (S. cerevisiae) | Info | XRRS2L | |
| **39** | 0.0007406 | 44.9 | 20.8 | 35.3 | 208363 s at | inositol polyphosphate-4- phosphatase, type I, 107kDa | Info | INPP4A | |
| **40** | 0.0008726 | 721 | 344.9 | 599.1 | 204588 s at | solute carrier family 7 (cationic amino acid transporter, and+ system), member 7 | Info | SLC7A7 | immunology |
| **41** | 0.0008942 | 277 | 140 | 259.8 | 201375 s at | protein phosphatase 2 (formerly 2A), catalytic subunit, beta isoform | Info | PPP2CB | |
| **42** | 0.0009282 | 31.2 | 63 | 54 | 207681 at | chemokine (C-X-C motif) receptor 3 | Info | CXCR3 | |
| **43** | 0.0009587 | 53.5 | 98.3 | 41.1 | 220024 s at | periaxin | Info | PRX | |
| **44** | 0.0009593 | 8684.9 | 15073.4 | 9366.9 | 1558678 s at | metastasis associated lung adenocarcinoma transcript 1 (non- coding RNA) | Info | MALAT1 | |
| **45** | 0.0009911 | 394.4 | 242.1 | 253.3 | 203247 s at | zinc finger protein 24 (KOX 17) | Info | ZNF24 | |

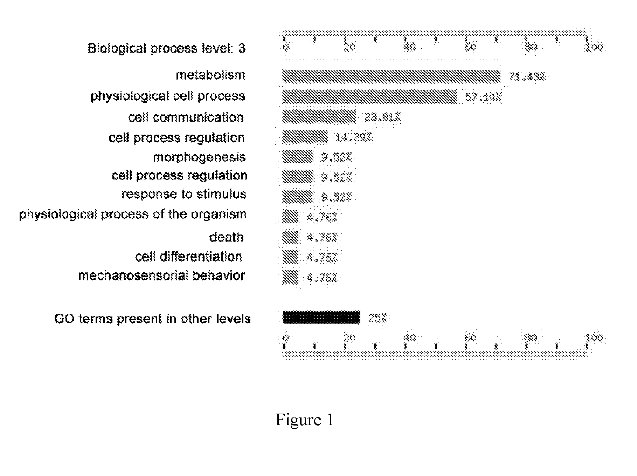

The distribution of these genes in Gene Ontology (GO) was not significantly different from that expected randomly (Figure 1).

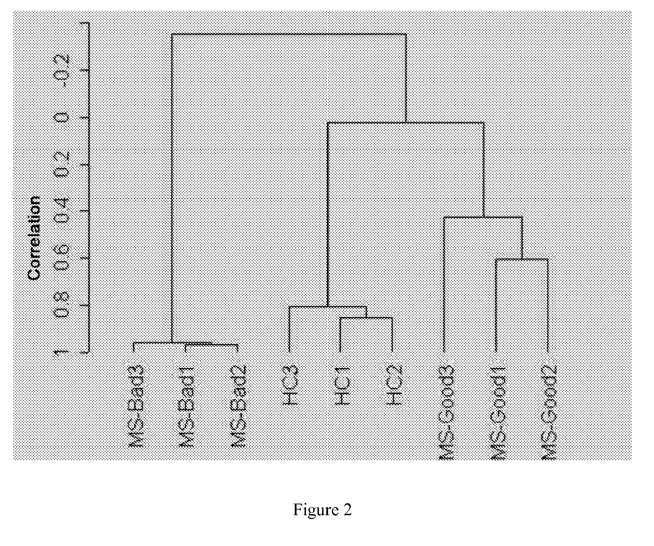

A cluster analysis of the samples was performed with these 45 genes and it was observed that 3 highly diagnostic reproducible clusters with a mean value of correlation between each cluster of approximately 0.6 (Figure 2) were formed.

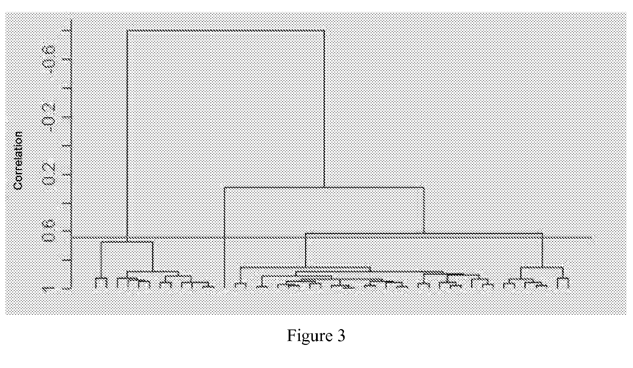

A cluster analysis was also performed with these 45 genes and it was observed that 4 clusters with a mean value of correlation of approximately 0.65 (Figure 3 and Table 4) were formed.

**Table 4: List of genes making up the 4 clusters.**

| Cluster | **Probe** | **Gene name** | **Gene symbol** |
|---|---|---|---|
| Cluster # | 11557278 s at | CDNA FLJ33199 fis, clone ADRGL2006377 | |
| | 207681 at | chemokine (C-X-C motif) receptor 3 | CXCR3 |
| | 216129 at | ATPase, Class II, tyep 9A | ATP9A |
| | 208050 s at | caspase 2, apoptosis-related cysteine protease (neural precursor cell expressed, developmentally down-regulated 2) | CASP2 |
| | 220024 s at | penaxin | PRX |
| | 23990 x at | | |
| | 1558678 s at | metastasis addociated lung adenocarcinoma transcript 1 (non-coding RNA) | MALAT1 |
| | 219805 at | hypothetical protein FLJ22965 | FLJ22965 |
| | 223226 x at | single stranded DNA binding protein 4 | SSBP4 |
| | 210436 at | chaperonin containing TCP1, subunit 8 (theta) | CCT8 |
| | 1570043 at | | |
| | 220366 at | epidymal spem binding protein 1 | ELSPBP1 |
| | | | |
| Cluster #2 | 215151 at | dedicaor of cytokinesis 10 | DOCK10< |
| | | | |
| Cluster #3 | 213659 at | zinc finger protein 75 (D8C6) | ZNF75 |
| | 218356 at | MSR2-like, magnesium homeostasis factor (S. cerevisiae) | MRS2L |
| | 204703 at | tetratricopeptide repeat domain 10 | TTC10 |
| | 240394 at | | |
| | 212310 at | C219-reactive peptide | FLJ39207 |
| | 205306 x at | kynurenine 3-monooxygenase (kynurenine 3-hydroxylase) | KMC |
| | 235401 s at | Fc receptor homolog expressed in B cells | FREB |
| | 217980 s at | mitochondrial ribosomal protein L16 | MRPL 16 |
| | 227268 at | PTD016 protein | LOC51136 |
| | 22138 s at | | |
| | 201302 at | annexin A4 | ANXA4 |
| | 36920 at | myotubularin 1 | MTM1 |
| | 225693 at | KIAA1340 protein | KIAA1340 |
| | 235177 at | similar to hepatocellular carcinoma-associated antigen HCA557b | LOC151194 |
| | 217301 x at | retinoblastoma binding protein 4 | RBP4 |
| | 201375 s at | protein phosphatase 2 (formerly 2a), catalytic subunit, beta isoform | PPP2CB |
| | 233558 s at | FLJ12716 protein | FLJ12716 |
| | 204588 s at | solute carrier family 7 (cationic amino acid transporter, y+ system), member 7 | SLC7A7 |
| | 205584 at | chromosome X open reading frame 45 | CXorf45 |
| | 208612 at | glucose regulated proetin, 58kDa | GRP58 |
| | 208363 s at | inositol polyphosphate-4-phosphate, type 1, 107kDa | NPP4A |
| | 232383 at | transcription factor EC | TFEC |
| | 201440 at | DEAD (Asp-Glu-Ala-Asp) box polypeptide 23 | DDX23 |
| | 20333 at | kinesin-associated protein 3 | KIFAP3 |
| | 204634 at | NIMA (never in mitosis gene a)-related kinase 4 | NEK4 |
| | | | |
| Cluster #4 | 203247 s at | zinc finger protein 24 (KOX 17) | ZNF24 |
| | 224945 at | BTB (POZ) domain containing 7 | BTBD7 |
| | 216944 s at | inositol 1,4,5-triphosphate receptor, type 1 | ITPR1 |
| | 227541 at | WD repeat domain 20 | WDR20 |
| | 229190 at | CDNA FLJ90295 fis, clone NT2PR2000240. | |
| | 23914 s at | synaprotagrin-like 2 | SYTL2 |
| | 235412 at | | |

For the purpose of complementing the number of genes which allow differentiating between each diagnosis, a class comparison test with a p value<0.001 between the bad and good prognosis, control and bad prognosis, control and good prognosis and control and multiple sclerosis classes (Tables 5, 6, 7 and 8), as well as a class comparison test between the three diagnoses with a p value<0.005 (Table 9), were performed.

**Table 5: Genes which presented significant differences between the bad and good prognosis classes with a p value<0.001.**

| | **P value** | **Bad prognosis** | **Good prognois** | **Difference ratio** | **Probe** | **Description** | **UG cluster** | **Gene symbol** | **Location** | **List of gnes** |
|---|---|---|---|---|---|---|---|---|---|---|
| **1** | 1.1e-06 | 10 | 22.6 | 0.442 | 210188 at | GA binding protein transcription factor, alpha subunit 60kDa | Hs.78 | GABPA | chr21q21.3 | |
| **2** | 4.6e-06 | 104 | 45.9 | 0.227 | 235401 s at | Fc receptor homolog expressed in B cells | Hs.2666331 | FREB | chr1q23.3 | |
| **3** | 8.2e-06 | 10 | 28.9 | 0.346 38.6 0.267 | 1554433 a at | zinc finger protein 146 | Hs.444223 | ZNF146 | chr19q13.1 | |
| **4** | 4.16e-05 | 10.3 | 38.6 | 0.267 | 222281 s at | | | | | |
| **5** | 4.31e-05 | 10 | 39.9 | 0.251 | 209602 s at | CATA binding proteins 3 | Hs.169946 | GATA3 | chr10p15 | gene_regulahon, immunology, misc, transcription |
| **6** | 4.86e-05 | 28.6 | 10.4 | 2.75 | 240990 at | | | | | |
| **7** | 0.0001008 | 24.9 | 10 | 2.49 | 223900 x at | | | | | |
| **8** | 0.0001254 | 10.7 | 42.7 | 0.251 | 205306 x at | kynurenine 3- monooxygnease (kynurenine 3- hydroxylase) | Hs.170129 | KMO | chr1q42- q44 | |
| **9** | 0.0001886 | 10.7 | 35.3 | 0.303 | 209421_at | mutS homolog 2, colon cancer nonpolyposis type 1 E.coli) | Hs.440394 | MSH2 | chr2p22- p21 | DNA_damage, tsonc |
| **10** | 0.0001993 | 10.4 | 33.8 | 0.308 | 240394 at | | | | | |
| **11** | 0.0002376 | 80.6 | 36.6 | 2.202 | 207389 at | glycoprotein 1b (platelet) alpha | Hs.1472 | GP1BA | chr17pter p12 | immunology |
| **12** | 0.0002527 | 11.2 | 27 | 0.415 | 200606 at | desmoplakin | Hs.349899 | DSP | chr6p24 | immunology |
| **13** | 0.0003294 | 25.3 | 10 | 2.53 | 219970 at | PDZ domain protein GIPC2 | Hs.13852 | GIPC2 | (chr1p31.1 | |
| **14** | 0.000331 | 10 | 23.9 | 0.418 | 217320 at | IgM rheumatoid factor RF-DI1, variable heavy chain | Hs.535538 | | | |
| **15** | 0.0003357 | 32.4 | 10 | 3.24 | 228367 at | heart alpha- kinase | Hs. 388674 | HAK | chr18q21.31 | |
| **16** | 0.0003577 | 135.6 | 62.4 | 2.173 | 223226 x at | single stranded DNA binding protein 4 | Hs.324618 | SSBP4 | chr19p13.1 | |
| **17** | 0.0004336 | 52.3 | 10.1 | 5.178 | 1560263 at | Hypothetical | Hs.169854 | SP192 | chr1p34.1 | |
| **18** | 0.000437 | 39.5 | 10.4 | | 3.798 242392 at protein | hypothetical MGC35130 | Hs.388746 | MGC35130 chrlp31.3 | | |
| **19** | 0.0004597 | 10 | 26.2 | 0.382 | 156387 a at | KIAA0826 | Hs.441602 | KIAA0826 | chr4p12 | |
| **20** | 0.0007879 | 12.9 | 32.9 | 0.392 | 221138 s at | | | | | |
| **21** | 0.0009951 | 105.9 | 243.9 | 0.434 | 223361 at | Chromosome 6 open reading | Hs.238205 | C6orfl15 | chr6q24.1 | |

**Table 6: Genes which presented significant differences between the control and bad prognosis classes with a p value<0.001.**

| | **P value** | **Healthy contr.** | **Bad prognosis** | **Difference ratio** | **Probe** | **Description** | **Annotation** | **UG cluster** | **Gene symbol** | **Location** | **List of genes** |
|---|---|---|---|---|---|---|---|---|---|---|---|
| **1** | 1.8e-06 | 37.7 | 10 | 3.77 | 239431 at | toll-like receptor adaptor molecule 2 | Info | Hs.534007 | TICAM2 | chr5q23.1 | |
| **2** | 5.7e-06 | 29.2 | 10 | 2.92 | 213659 at | zinc finger protein 75 (D8C6) | Info | Hs.131127 | ZNF75 | chrxq26.3 | |
| **3** | 1.15e-05 | 54.9 | 10 | 5.49 | 239842 x at | | Info | | | | |
| **4** | 2.96e-05 | 14.4 | 54.1 | 0.266 | 1559049 a at | CDNA FLJ30371 fis, clone BRACE2007836 | Info | Hs.154483 | | | |
| **5** | 4.08e-05 | 37.4 | 10 | 3.74 | 2212 s at | SH2 domain containing phosphatase anchor protein 1 anchor protein 1 /// SH2 domain | Info | Hs.194976 | SPAP1 | chr1q21 | |
| **6** | 6.1e-05 | 34.3 | 10 | 3.43 | 224163 s at | DNA methyltransferase 1 associated protein 1 | Info | Hs.8008 | DMAP1 | chr1p34 | |
| **7** | 0.0001299 | 37.8 | 10.7 | 3.533 | 205306 x at | kynurenine 3- monooxygenase (kynurenine 3- hydroxylase) | Info | Hs.170129 | KMO | chr1q42-q44 | |
| **8** | 0.000135 | 19.6 | 56.4 | 0.348 | 219805 at | hypothetical protein FLJ22965 | Info | Hs.248572 | FLJ22965 | chrxq23 | |
| **9** | 0.0002222 | 567.1 | 147.5 | 3.845 | 212192 at | potassium channel tetramerisation containing 12 | Info | Hs.109438 | KCTD12 | chr13q22.3 | |
| **10** | 0.0002518 | 10 | 38.6 | 0.259 | 1559976 at | CDNA FLJ36082 fis, clone TEST1201998 | Info | Hs.322679 | | | |
| **11** | 0.0002543 | 21.1 | 56.1 | 0.376 | 1556024 at | SPRY domain- containing SOCS box protein SSB-3 | Info | Hs.7247 | SSB3 | chr16p13.3 | |
| **12** | 0.000286 | 446.6 | 164.6 | 2.713 | 212033 at | RNA binding motif protein 25 | Info | Hs.197184 | RBM25 | chr14q24.3 | |
| **13** | 0.0003111 | 10.9 | 37.4 | 0.291 | 1569013 s at | Hypothetical Protein similar to KIAA0187 gene | Info | Hs.356397 | LOC96610 | chr22q11.22 | |
| **14** | 0.0003151 | 27.9 | 10 | 2.79 | 234445 at | chromosome 6 open reading frame 12 | Info | Hs.302037 | C6orf12 | chr6p21.33 | |
| **15** | 0.0003357 | 10 | 32.4 | 0.309 | 228367 at | heart alpha-kinase | Info | Hs.388674 | HAK | chr18q21.31 | |
| **16** | 0.0003458 | 70.5 | 5.316 | | 228030 at | | Info | | | | |
| **17** | 0.0003604 | 46 | 10 | 4.6 | 232383 at | | Info | | | | |
| **18** | 0.0004024 | 10.2 | 33.6 | 0.304 | 213965 s at | chromodomain helicase DNA binding protein 5 | Info | Hs.388126 | CHD5 | chr1p36.31 | |
| **19** | 0.0004329 | 11.2 | 42.7 | 0.262 | 153063 at | Homo sapiens, clone IMAGE:5164544 | Info | Hs.385801 | | | |
| **20** | 0.0004544 | 11.1 | 31.8 | 0.349 | 242251 at | Info | | | | | |
| **21** | 0.0004575 | 137.4 | 31.8 911.8 | 0.349 0.151 | 205950 s at | carbonic anhydrase 1 | Info | Hs.23118 | CA1 | chr8q13-q22.1 | Immunology |
| **22** | 0.0004637 | 36.8 | 10.4 | 3.538 | 235401 s at | Fx receptor homolog expressed in B cells | Info | Hs.266331 | FREB | chr1q23.3 | |
| **23** | 0.0004737 | 40.5 | 10 | 4.05 | 235412 at | | Info | | | | |
| **24** | 0.0005659 | 11.5 | 39.9 | 0.288 | 203683 s at | vascular endothelial growth factor B | Info | Hs.78781 | VEGEB | chr11q13 | Angiogenesis, misc |
| **25** | 0.0006652 | 46.7 | 165.4 | 0.282 | 233371 at | ATP-binding cassette, sub-family C (CFTR/MRP), | Info | Hs.366575 | ABCC13 | chr21q11.2 | |
| **26** | 0.0006666 | 45.3 | 10.5 | 4.314 | 235177 at | similarto hepatocellular carcinoma-associated HCA557b | Info | Hs.352294 | LOC151194 | chr2q33.3 | |
| **27** | 0.0006781 | 704.4 | 182.4 | 3.862 | 213566 at | ribonuclease, RNase A family, k6 /// ribonuclease, RNase A family, k6 | Info | Hs.23262 | RNASE6 | chr14q11.2 | |
| **28** | 0.0007095 | 10.9 | 29.7 | 0.367 | 239471 at | Leucine rich repeat containing 28 | Info | Hs.390622 | LRRC28 | chr15q26.3 | |
| **29** | 0.0008529 | 96.8 | 30.2 | 3.205 | 222558 s at | FLJ12716 protein | Info | Hs.443240 | FLJ12716 | chr4q35.1 | |
| **30** | 0.0008637 | 23.1 | 67.7 | 0.341 | 213779 at | EMI domain containing 1 | Info | Hs.289106 | EMIDI | chr22q12.2 | |
| **31** | 0.0009255 | 25.6 | 10.3 | 2.485 | 222412 s at | signal sequence receptor, gamma (translocon-associated protein gamma) | Info | Hs.28707 | SSR3 | chr3q25.31 | |
| **32** | 0.0009687 | 10.5 | 29.9 | 0.351 | 233538 s at | | Info | | | | |

**Table 7: Genes which presented significant differences between the control and good prognosis classes with a p value<0.001.**

| | **P value** | **Healthy controls** | **Good prognosis** | **Difference ratios** | **Probe** | **Description** | **UG cluster** | **Gene symbol** | **Location** | **List of genes** |
|---|---|---|---|---|---|---|---|---|---|---|
| **1** | 7.55e-05 | 65.6 | 30.3 | 2.165 | 228738 at | (hypothetical protein MGC25181 | Hs.511975 | MGC25181 | chr2p25.3 | |
| **2** | 0.0002827 | 11.5 | 37.4 | 0.307 | 240486 at | | | | | |
| **3** | 0.000318 | 49.8 | 11.2 | 4.446 | 227233 at | tetraspan 2 | Hs.2348463 | TSPAN-2 | chr1p13.1 | |
| **4** | 0.0004631 | 264.3 | 128.5 | 2.057 | 203231 s a | ataxin 1 | Hs.434961 | ATXN1 | chr6p23 | |
| **5** | 0.0005605 | 10.4 | 23.9 | 0.435 | 217320 at | IgM rheumatoid factor RF-DI1, variable heavy chain | Hs.535538 | | | |
| **6** | 0.0007329 | 63.8 | 134.2 | 0.475 | 230566 at | hypothetical protein FLJ35801 | Hs.52184 | FLJ35801 | chr22q12.2 | |
| **7** | 0.0008633 | 10.5 | 25.3 | 0.415 | 1553313 s at | solute carrier family 5 (inositol transporters), member 3 | Hs.534372 | SLC5A3 | chr21q22.12 | |
| **8** | 0.0009997 | 10 | 24.8 | 0.403 | 1556589 at | CDNA FLJ25645645 fis, clone SYN00113 | Hs.368190 | | | |
| **9** | 0.0012821 | 41.4 | 16.7 | 2.479 | 239801at | Hypothetical LOC400523 | Hs.534916 | | chr16p11.2 | |
| **10** | 0.0017972 10.5 | 10.5 | 30.5 | 0.344 | 228559 at | CDNA clone IMAGE:6043059, partial cds | Hs.55028 | | | |

**Table 8: Genes which presented significant differences between the control and multiple sclerosis classes with a p value<0.001.**

| | **P value** | **Healthy controls** | **MS patients** | **Difference ratio** | **Probe** | **Description** | **Annotation** | **UG cluster** | **Gene symbol** | **Location** | **List of genes** |
|---|---|---|---|---|---|---|---|---|---|---|---|
| **1** | p<le-07 | 20.6 | 10 | 2.06 | 229190 at | | Info | | | | |
| **2** | 2.99e-05 | 82.2 | 36.9 | 2.228 | 216944 s at | inositol 1,4,5-triphophate receptor, type | Info | Hs.149900 | ITPR1 | chr3p26-p25 | |
| **3** | 0.0001192 | 10.8 | 31.5 | 0.343 | 1553491 at | kinase suppressor of Ras-2 | Info | Hs.375836 | KSR2 | chr12q24.22- q24.23 | |
| **4** | 0.0001509 | 32.9 | 72.4 | 0.454 | 228247 at | SLIT-ROBORho GTPase activating protein 1 /// Similar to FRG1 protein (FSHD region gene 1 protein) | Info | Hs.446528 /// Hs.450763 | SRGAP1 ///MGC721 04 | chr12q14.2 /// ch20q11.1 | |
| **5** | 0.0002005 | 11.5 | 36.1 | 0.319 | 203683 s at | vascular endothelial growth factor B | Info | Hs.78781 | VEGFB | chr11q13 | Angiogene sis, misc |
| **6** | 0.0002331 | 118.2 | 50.2 | 2.355 | 213119 at | solute carrier family 36 (proton/amino acid symporter), member 1 | Info | Hs.409314 | SLC36A1 | chr5q33.1 | |
| **7** | 0.0002652 | 23.8 | 59.5 | 0.4 | 219380 x at | polymerase (DNA directed), eta | Info | H.s439153 | POLH | chr6p21.1 | |
| **8** | 0.0003315 | 177.3 | 458.2 | 0.387 | 214041 x at | | Info | | | | |
| **9** | 0.000344 | 15.1 | 63.8 | 0.237 | 210910 s at | POM (POM121 homolog, rat) and ZP3 fusion | Info | Hs.296380 | POMZP3 | chr7q11.23 | |
| **10** | 0.0004374 | 446.6 | 171.5 | 2.604 212033 | at | RNA binding motif protein 25 | Info | Hs.197184 | RBM25 | chr14q24.3 | |
| **11** | 0.000703 | 13 | 39.3 | 0.331 | 21239 x at | | Info | | | | |
| **12** | 0.0007383 | 11.3 | 25.4 | 0.445 | 220681 at | | Info | | | | |
| **13** | 0.0008143 | 11.4 | 30.4 | 0.375 | 1563715 at | mRNA; cDNA DKFZp761B0 221 (from DKFZp761B0 221) | Info | Hs.541764 | | | |
| **14** | 0.0008227 | 25.6 | 49.4 | 0.518 | 231812 x at | | Info | | | | |
| **15** | 0.0008812 | 236.4 | 105 | 2.251 | 219242 at | centrosome protein Cep63 | Info | Hs.443301 | Cep63 | chr3q22.1 | |
| **16** | 0.0009018 | 77.9 | 30.7 | 2.537 | 206618 at | interleukin 18 interleukin 18 receptor 1 | Info | Hs.159301 | IL18R1 | chr2q12 | Immunolo |

**Table 9: Genes which presented significant differences between the three classes (control, bad prognosis and good prognosis) with a p value<0.005.**

| | **P value** | **Healthy controls** | **Bad prognosis** | **Good prognosis** | **Probe** | **Description** | **Annotation** | **Gene symbol** | **List of genes** |
|---|---|---|---|---|---|---|---|---|---|
| **1** | 2e-07 | 15.9 | 39.7 | 26 | 1557278 s at clone | CDNA FLJ33199 fis, ADRGL2006377 | Info | | |
| **2** | 3e-07 | 20.6 | 10 | 10 | 229190 at | CDNA FLJ90295 fis, clone FLJ90295 fis, NT2RP2000240. | Info | | |
| **3** | 7.4e-06 | 37.8 | 10.7 | 42.7 | 205306 x at | kynurenine 3-monooxygenase (kynurenine 3-hydroxylase) | Info | KMO | |
| **4** | 1.07e-05 | 59.8 | 36.3 | 35.4 | 227541 at | WD repeat domain 20 | Info | WDR20 | |
| **5** | 1.19e-05 | 36.8 | 10.4 | 45.9 | 235401 s at | Fc receptor homolog expressed in B cells | Info | FREB | |
| **6** | 2.07e-05 | 61 | 135.6 | 62.4 | 223226 x at | single stranded DNA binding protein 4 | Info | SSBP4 | |
| **7** | 2.13e-05 | 10.6 | 22.6 | 10 | 210436 at | chaperonin containing TCP1 subunit 8 (theta) | Info | CCT8 | |
| **8** | 6.14e-05 | 117.5 | 69.7 | 80.6 | 224945 at | BTB (POZ) domain containing 7 | Info | BTBD7 | |
| **9** | 9.61e-05 10 | 20.3 | | 10.7 | 1570043 at | | Info | | |
| **10** | 0.0001169 | 19.6 | 56.4 | 28.8 | 219805 at | hypothetical (protein FLJ22965 | Info | FTJ22965 | |
| **11** | 0.0002011 | 24.6 | 10.4 | 33.8 | 240394 at | | Info | | |
| **12** | 0.0002318 | 46 | 10 | 25.9 | 232383 at | transcription factor EC | Info | TFEC | |
| **13** | 0.0002505 | 31.6 | 12.9 32.9 | | 221138 s at | | Info | | |
| **14** | 0.0002614 | 116.8 | 52.7 | 70.8 | 232914 s at | synaptotagmin-like 2 | Info | SYTL2 | |
| **15** | 0.0002713 | 41.4 | 11.8 | 35 | 204634 at | NIMA (never in mitosis gene a)-related kinase 4 | Info | NEK4 | |
| **16** | 0.0002741 | 127.5 | 62.9 | 133.1 | 201302 at | annexin A4 | Info | ANXA4 | |
| **17** | 0.0003003 | 82.2 | 35.7 | 38.2 | 216944 s at | inositol 1,4,5-triphosphate receptor, type 1 | Info | ITPR1 | |
| **18** | 0.0003018 | 40.5 | 10 | 21.2 | 235412 at | | Info | | |
| **19** | 0.0003059 | 66 | 40.2 | 61.5 | 203333 at | kinesin-associated protein 3 | Info | KIPAP3 | |
| **20** | 0.0003135 | 10.8 | 10.5 | 25 | 215151 at | dedicator of cytokinesis 10 | Info | DOCK10 | |
| **21** | 0.0003224 | 96.8 | 30.2 | 81.1 | 233558 s at | FLJ12716 protein | Info | FLJ12716 | |
| **22** | 0.00037324 | 155.2 | 80.9 | 158.1 | 217301 x at | retinoblastoma binding protein 4 | Info | RBBP4 | |
| **23** | 0.0003848 | 20.3 | 43.6 | 16.3 | 208050 s at | caspase 2, apoptosis-related cysteine protease (neural precursor cell expressed, developmentally down-regulated | Info | CASP2 | apoptosis, immunology |
| **24** | 0.0004095 40.1 | 40.1 | 17 | 42.7 | 36920 at | myotubularin 1 | Info | MTM1 | |
| **25** | 0.0004232 | 26.2 | 10 | 23.7 | 225963 at | KIAA1340 protein | Info | KIAA1340 | |
| **26** | 0.0004529 | 34.5 | 11.6 | 36.8 | 212310 at | C219-reactive peptide | Info | FLJ39207 | |
| **27** | 0.0004554 | 45.3 | 10.5 | 34.3 | 235177 at | similar to hepatocellular carcinoma- antigen HCA557b | Info | LOC151194 | |
| **28** | 0.0004832 | 61.7 | 21.2 | 67 | 227268 at | PTD016 protein | Info | LOC51136 | |
| **29** | 0.000489 | 422 | 205.2 | 370.8 | 208612 at | glucose regulated protein, 58kDa | Info | GRP58 | |
| **30** | 0.0005587 | 29.2 | 10 | 36.7 | 213659 at | zinc finger protein 75 (D8C6) | Info | ZNF75 | |
| **31** | 0.0005844 147.3 | 147.3 | 88.7 | 152.8 | 217980 s at | mitochondrial ribosomal protein L16 | Info | MRPL16 | |
| **32** | 0.0005845 68.7 | 68.7 | 21.2 | 45.6 | 205584 at | chromosome X open reading frame 45 | Info | CXorf45 | |
| **33** | 0.0006039 | 11.1 | 49 | 11.9 | 220366 at | epididymal sperm binding protein 1 | Info | ELSPBP1 | |
| **34** | 0.0006295 | 140.1 | 75.7 | 134.7 | 201440 at | DEAD (Asp-Glu-Ala-Asp) box polypeptide 23 | Info | DDX23 | |
| **35** | 0.0006427 | 12 | 24.9 | 10 | 239900x at | | Info | | |
| **36** | 0.0006527 | 52.3 | 28.5 | 63 | 204703 at | tetratricopeptide repeat domain 10 | Info | TTC10 | |
| **37** | 0.0007041 | 16.7 | 40.1 | 12.1 | 216129 at | ATPase, Class II, type 9A | Info | ATP9A | |
| **38** | 0.0007168 | 42.2 | 25.1 | 56.5 | 218536 at | MRS2-like magnesium homeostasis factor (S. cerevisiae) | Info | MRS2L | |
| **39** | 0.0007406 | 44.9 | 20.8 | 35.3 | 208363 s at | inositol polyphosphate-4-phosphatase, type I, 107kDa | Info | INPP4A | |
| **40** | 0.0008726 | 721 | 344.9 | 599.1 | 204588 s at | solute carrier family 7 (cationic amino acid transporter, and+ system), member 7 | Info | SLC7A7 | immunology |
| **41** | 0.0008942 | 277 | 140 | 259.8 | 201375 s at | protein phosphatase 2 (formerly 2A), catalytic subunit, beta isoform | Info | PPP2CB | |
| **42** | 0.0009282 | 31.2 | 63 | 54 | 207681 at | chemokine (C-X-C motif) receptor | Info | CXCR3 | |
| **43** | 0.0009587 | 53.5 | 98.3 | 41.1 | 220024 s at | periaxin | Info | PRX | |
| **44** | 0.0009593 | 8684.9 | 15073.4 | 9366.9 | 1558678 s at | metastasis associated lung adenocarcinoma transcript 1 (non-coding RNA) | Info | MALAT1 | |
| **45** | 0.0009911 | 394.4 | 242.1 | 253.3 | 203247 s at | zinc finger protein 24 (KOX 17) | Info | ZNF24 | |
| **46** | 0.0010197 | 11.5 | 28.1 | 18 | 244340 x at | | Info | | |
| **47** | 0.0010259 | 117.3 | 70 | 147.1 | 213848 at | dual specificity phosphatase 7 | Info | DUSP7 | |
| **48** | 0.0010309 | 11.3 | 25.1 | 10.1 | 1559441 s at subfamily V, | cytochrome P450, family 4, polypeptide 2 | Info | CYP4V2 | |
| **49** | 0.0010448 29.4 | 29.4 | 10.7 | 35.3 | 209421 at | mutS homolog 2, colon cancer, nonpolyposis type 1 (E. coli) | Info | MSH2 | DNA_damage, tsonc |
| **50** | 0.0010622 | 19 | 10 | 21.4 | 218884 s at | hypothetical protein FLJ13220 | Info | FLJ13220 | |
| **51** | 0.0010626 | 118.2 | 54.7 | 46.1 | 213119 at | solute carrier family 36 (proton/amino acid symporter), member 1 | Info | SLC36A1 | |
| **52** | 0.0010771 | 136.4 | 67.7 | 139.2 | 228234 at | toll-like receptor adaptor molecule 2 | Info | TICAM2 | |
| **53** | :0.0010849 | 10.8 | 32.8 | 30.3 | 1553491 at | kinase suppressor of Ras-2 | Info | KSR2 | |
| **54** | 0.001096 | 180.3 | 89.4 | 149.4 | 218098 at | ADP-ribosylation factor guanine nucleotide- exchange factor 2 (brefeldin A-inhibited) | Info | ARFGEF2 | |
| **55** | 0.0011018 | 11.5 | 39.9 | 32.7 | 203683 s at | vascular endothelial growth factor B | Info | VEGFB | angiogenesis, misc |
| **56** | 0.0011241 | 12.6 | 36.8 | 11.3 | 214997 at | golgi autoantigen, golgin subfamily a, 1 | Info | GOLGA1 | |
| **57** | 0.0011297 33.7 | 33.7 | 15.6 | 46 | 213063 at | nuclear protein UKp68 | Info | FLJ11806 | |
| **58** | 0.0011697 | 203.2 | 112.4 | 104.3 | 213906 at | v-myb myeloblastosis viral oncogene homolog (avian)-like 1 | Info | MYBL1 | tsonc |
| **59** | 0.0011949 | 35.2 | 10 | 22.7 | 204113 at | CUG triplet repeat, RNA binding protein 1 | Info | CUGBP1 | |
| **60** | 0.0012042 | 111.9 | 51.7 | 61.5 | 229510 at | testes development-related NYD- SP21 | Info | NYD-SP21 | |
| **61** | 0.0012117 | 150.6 | 56.3 | 111.9 | 201816 s at | glioblastoma amplified sequence | Info | GBAS | |
| **62** | 0.0012122 | 235.4 | 35.1 | 166.2 | 212956 at | KIAA0882 protein | KIAA0882 Info | KIAA0882 | |
| **63** | 0.0012465 | 32.9 | 75.1 | 69.7 | 228247 at | zinc finger protein 542 /// similar to FRG1 protein (FSHD region gene 1 protein) | Info | ZNF542 /// MGC72104 | |
| **64** | 0.0012784 | 10.5 | 41 | 10 | 203934 at | kinase insert domain receptor (a type III receptor tyrosine kinase) | Info | KDR | ansiosenesis, cell_cycle, cell_signaling, immunology, signal_transduc tion |
| **65** | 0.0013104 | 445.7 | 221 | 326.1 | 203567 s at | tripartite motif-containing 38 | Info | TRIM38 | |
| **66** | 0.0013985 | 54.9 | 10 | 34.2 | 239842 x at | | Info | | |
| **67** | 0.0014599 | 22.6 | 49.9 | 36.6 | 219089 s at | zinc finger protein 576 | Info | ZNF576 | |
| **68** | 0.0014674 | 62.3 | 24.1 | 47.7 | 238601 at | | Info | | |
| **69** | 0.0014801 | 16.1 | 38.6 | 23.3 | 32540 at | | Info | | |
| **70** | .0015065 | 11.1 | 29.3 | 10.3 | 220791 x at | sodium channel, voltage-gated, type XI, alpha | Info | SCN11A | |
| **71** | 0.0015118 | 38.4 | 12.1 | 50.6 | 212533 at | WEE1 homolog (S. pombe) | Info | WEE1 | immunology |
| **72** | 0.0015299 | 68.4 | 15.1 | 64.8 | 227856 at | hypothetical protein FLJ39370 | Info | FLJ39370 | |
| **73** | 0.0015713 | 126.1 | 57 | 119.8 | 200950 at | actin related protein 2/3 complex, subunit 1A, 41kDa | Info | ARPC1A | |
| **74** | 0.0015729 | 38.4 | 10.3 | 38.6 | 222281 s at | 1A,4 1kDa | Info | | |
| **75** | 0.0015784 | 37.5 | 10 | 28.9 | 1554433 a at | zinc finger protein 146 | Info | ZNF146 | |
| **76** | 0.0015902 | 33.8 | 11 | 26 | 1553225 s at | zinc finger protein 75 (D8C6) | Info | ZNF75 | |
| **77** | 0.0016105 | 113.7 | 49 | 97.8 | 211537 x at | mitogen-activated protein kinase 7 | Info | MAP3K7 | |
| **78** | 0.0016961 115.2 | 115.2 | 119.4 | 72.5 | 204046 at | phospholipase C, beta 2 | Info | PLCB2 | |
| **79** | 0.0017071 | 22.5 | 10 | 29.2 | 213132 s at | malonyl-COA:acyl carrier protein transacylase, mitochondrial | Info | MT | |
| **80** | 0.0017268 | 10.7 | 34 | 21.3 | 1554106 at | amyotrophic (lateral sclerosis 2 (juvenile) chromosome region, candidate | Info | ALS2CR16 | |
| **81** | 0.0017475 | 12 | 38 | 19.1 | 1557292 a at | mucolipin 3 | Info | MCOLN3 | |
| **82** | 0.0017591 | 105.4 | 234.7 | 142.1 | 23917 at | | Info | | |
| **83** | 0.0017977 33.5 | 33.5 | 61.2 | 28 | 1557961 s at | | Info | | |
| **84** | 0.0018152 | 15.1 | 72.7 | 55.9 | 21091 s at | POM(POM121 homolog, rat) and ZP3 fusion | Info | POMZP3 | |
| **85** | 0:0.0018156 | 13.5 | 10 | 30.2 | 220643 s at | Fas apoptotic inhibitory molecule | Info | FAIM | |
| **86** | 0.001832 | 41.1 | 93.6 | 50.1 | 215583 at | KIAA0792 gene product | Info | KIAA0792 | |
| **87** | 0.0018501 | 10 | 33.1 | 16.8 | 204179 at | myoglobin | Info | MB | |
| **88** | 0.0018544 | 27.5 | 10.9 | 28.2 | 1555201 a at | chromosome 6 open readingframe 96 | Info | C6orf96 | |
| **89** | 0.0018583 | 116.2 | 59.2 | 88.2 | 239243 at | | Info | Info | |
| **90** | 0.0018584 | 89.7 | 40.4 | 78.7 | 225161 at | mitochondrial elongation factor G1 | Info | EFCl | |
| **91** | 0.0019076 341.6 | 341.6 | 177.2 | 341.6 | 211675 s at | 1-mfa do main-containing protein /// 1-mfa containing protein | Info | HIC | |
| **92** | 0.0019228 | 195.8 | 99.8 | 191.4 | 227319 at | chromosome 16 open reading frame 44 | Info | C160rf44 | |
| **93** | 0.0020078 | 28.5 | 24.8 | 50.5 | 213149 at | dihydrolipoamide acetyltransferase (E2 component of pyruvate dehydrogenase complex) | Info | DLAT | |
| **94** | 0.0020628 | 226.3 | 89.5 | 169.2 | 209203 s at | bicaudal D homolog 2 (Drosophila) | Info | BICD2 | |
| **95** | 0.0020776 | 10.7 | 24.1 | 11.6 | Hypothetical 1560204 at | hypothetical (protein LOC284958 | Info | | |
| **96** | 00.0021699 | 34.5 | 84.5 | 45.3 | 202383 at | Jumonji, AT rich interactive domain 1C (RBP2-like) | Info | JARIDIC | |
| **97** | 0.002178 | 33.3 | 73.1 | 42.3 | 213681 at | cysteine and histidine rich 1 | Info | CYHR1 | |
| **98** | 0.0022017 | 11.9 | 25.3 | 10 | 219970 at | pDZ domain protein GIPC2 | Info | GIPC2 | |
| **99** | 0.0022244 | 58.4 | 80.6 | 36.6 | 207389 at | glycoprotein 1b (platelet), alpha polypeptide | Info | GP1BA | immunology |
| **100** | 0.0022335 | 205.6 | 94 | 171 | 218715 at | hepatocellular carcinoma- associatedantigen 66 | Info | HCA66 | |
| **101** | 0.0022357 | 10 | 23.7 | 18 | 235462 at | | Info | | |
| **102** | 0.0022369 | 23.8 | 61.2 | 57.8 | 219380 x at | polymerase (DNA directed), eta | Info | POLH | |
| **103** | 0.0022546 | 205.8 | 91.6 | 185.3 | 203922 s at | cytochrome b-245, beta polypeptide (chronic granulomatous disease) | Info | CYBB | immunology |
| **104** | 0.0022665 | 10.1 | 26.1 | 15.4 | 233246 at | HSPC090 mRNA, partial cds | Info | | |
| **105** | 0.0023625 | 209.6 | 18 | 75.5 | 205321 at | eukaryotic translation initiation factor 2, subunit 3 gamma, 52kDa | Info | EIF2S3 | |
| **106** | 0.0024026 | 34.7 | 69.4 | 42.2 | 243051 at | | Info | | |
| **107** | 0.0024423 | 82.3 | 24.9 | 667.6 | 222646 s at | ERO1-like(S. cerevisiae) | Info | ERO1L | |
| **108** | 0.0024457 | 13 | 28.6 | 10.4 | 240990 at | | Info | | |
| **109** | 0.0024576 | 183.4 | 78.7 | 161.3 | 201301 s at | annexin A4 | Info | ANXA4 | |
| **110** | 0.0025048 | 43.3 | 18.2 | 47.8 | 205427 at | zinc finger protein 354A | Info | ZNF354A | |
| **111** | 0.0025413 | 13 | 34.2 | 45.1 | 217239 x at | | Info | | |
| **112** | 0.0025506 | 49.5 | 15.1 | 37.2 | 207968 s at | MADS box transcription enhancer factor 2, polypeptide C (myocyte enhancer factor 2C) | Info | MEF2C | |
| **113** | 0.0025715 | 10.1 | 15.3 | 19.6 | 232962 x at | CDNA FLJ11549 fis, clone HEMBA1002968 | Info | | |
| **114** | 0.0025796 | 31.2 | 13.1 | 26.3 | 204995 at | cyclin-dependent kinase 5, regulatory subunit 1 (p35) | Info | cDK5R1 | |
| **115** | 0.0026228 | 12.7 | 41.9 | 12.9 | 204042 at | WAS protein family, member 3 | Info | WASF3 | |
| **116** | 0.0026255 | 24.9 | 12.7 | 29.6 | 231913 s at | c6.1A | Info | C6.1A | |
| **117** | 0.0027291 | 57 | 67.7 | 114 | 201614 s at | RuvB-like 1 (E. coli) | Info | RUVBL1 | |
| **118** | 0.0027564 | 357.6 | 164.7 | 296.4 | 20747 x at | calpastatin | Info | CAST | |
| **119** | 0.0027577 | 13.8 | 52.3 | 32.4 | 1553647 at | chromodomain protein, E-like 2 | Info | CDYL2 | |
| **120** | 0.0028454 | 11.4 | 25.8 | 16.7 | 231985 at | flavoprotein oxidoreductase MICAL3 | Info | MICAL3 | |
| **121** | 0.0028521 | 177.3 | 458.5 | 458 | 214041 x at | | Info | | |
| **122** | 0.0028675 | 115.5 | 73.7 | 128.2 | 203630 s at | component of oligomeric golgi complex 5 | Info | COG5 | |
| **123** | 0.0029057 | 24.6 | 13.6 | 36.9 | 227751 at | programmed cell death 5 | Info | PDCD5 | |
| **124** | 0.0029409 | 541.3 | 310.4 | 529.5 | 208819 at | member RAB8A, member RAS oncogene family | Info | RAB8A | |
| **125** | 0.0029651 | 319.5 | 97.6 | 235.8 | 227260 at | | Info | | |
| **126** | 0.0029731 | 196.8 | 114.1 | 203.9 | 218604 at | integral inner nuclear membrane protein | Info | MAN1 | |
| **127** | 0.0029877 | 14 | 34.9 | 12.7 | 206654 s at | polymerase RNA) III (DNA directed) polypeptides G (32kD) | Info | POLR3G | |
| **128** | 0.0029972 | 129.2 | 56.3 | 116.1 | 224876 at | hypothetical protein FLJ37562 | Info | FLJ37562 | |
| **129** | 0.0030549 | 11.4 | 34.4 | 26.9 | 1563715 at | | Info | | |
| **130** | 0.0031053 | 206.5 | 82.2 | 151.2 | 241993 x at | | Info | | |
| **131** | 0.0031432 12 | 12 | 22.2 | 10 | 233086 at | chromosome 20 open reading frame 106 | Info | C20orf106 | |
| **132** | 0.0031432 | 92.3 | 88.9 | 42.9 | 226018 at | hypothetical protein Ellsl | Info | Ellsl | |
| **133** | 0.003172 | 29 | 11.1 | 25.4 | 231975 s at | hypothetical protein FLJ35954 | Info | FLJ35954 | |
| **134** | 0.0031828 | 13.1 | 39.5 | 10.4 | 242392 at | hypothetical protein MGC35130 | Info | MGC35130 | |
| **135** | 0.0032055 | 41.4 | 14.7 | 36.4 | 220201 at | membrane associated DNA binding protein | Info | MNAB | |
| **136** | 0.0032743 | 446.6 | 164.6 | 178.8 | 212033 at | RNA binding motif protein 25 | Info | RBM25 | |
| **137** | 0.0033044 82.6 | 82.6 | 132.4 | 72 | 215504 x at | Clone 25061 mRNA sequence | Info | | |
| **138** | 0.003329 | 145.3 | 494.4 | 222 | 224321 at | transmembrane protein with EGF-like and two follistatin- like domains 2 /// transmembrane protein with EGF-like and two follistatin- like domains 2 | Info | TMEFF2 | |
| **139** | 0.0033665 | 71.3 | 20.2 | 42 | 225922 at | KIAA1450 protein | Info | KIAA1450 | |
| **140** | 0.0033774 | 171.7 | 76.7 | 175.5 | 201259 s at | synaptophysin-like protein | Info | SYPL | |
| **141** | 0.0033795 | 50.5 | 19.5 | 39.1 | 225754 at | adaptor related protein complex 1, gamma 1 subunit | Info | AP1G1 | |
| **142** | 0.0034069 | 11.7 | 40.3 | 27.9 | 243343 at | | Info | | |
| **143** | 0.003445 | 171.9 | 61.3 | 144.7 | 201711 x at | RAN binding protein 2 | Info | RANBP2 | gene_regulatio n, transcription |
| **144** | 0.0034662 26.6 | 26.6 | 15.8 | 34.7 | 228561 at | | Info | | |
| **145** | 0.0035038 | 36.8 | 76 | 52.7 | 1552646 at | interleukin 11 receptor, alpha | Info | IL11RA | immunology |
| **146** | 0.0035692 | 65.1 | 39 | 64.7 | 217043 s at | mitofusin 1 | Info | MFN1 | |
| **147** | 0.0036899 30.6 | 30.6 | 11.5 | 29.9 | 219608 s at | F-box protein 38 | Info | FBXO38 | |
| **148** | 0.0037154 | 130.1 | 53.4 | 149.4 | 231736 x at | microsomal glutathione S- transferase 1 | Info | MGST1 | pharmacology |
| **149** | 0.0037166 | 62.4 | 23.3 | 51.9 | 226894 at | | Info | | |
| **150** | 0.0037726 | 175.8 | 88.5 | 133.8 | 222000 at | hypothetical protein LOC339448 | Info | LOC339448 | |
| **151** | 0.0037966 | 325.1 | 103 | 234 | 221841 s at | Kruppel-like factor 4 (gut) | Info | KLF4 | |
| **152** | 0.0038994 | 60.9 | 31.9 | 60.1 | 223404 s at | chromosome 1 open reading frame 25 | Info | C1orf25 | |
| **153** | 0.0039171 | 52.7 | 16.8 | 43.6 | 210635 s at | kelch-like ECT2 interacting protein | Infb | KIEIP | |
| **154** | 0.003963 | 33.8 | 34.7 | 14.3 | 222426 at | mitogen- activated protein kinase associated protein 1 | Info | MAPKAP1 | |
| **155** | 0.0039777 58.7 | 58.7 | 117.2 | 96.7 | 236346 at | | Info | | |
| **156** | 0.0040561 | 16.1 | 33.1 | 22.1 | 216261 at | integrin, beta 3 (platelet glycoprotein IIIa, antigen CD61) | Info | ITGB3 | cell signaling, immunology, metastasis |
| **157** | 0.0040985 | 13.8 | 28.3 | 19.7 | 241695 s at | | Info | | |
| **158** | 0.0041185 | 112.2 | 45.9 | 97.4 | 238017 at | potassium channel tetramerisation domain containing 6 | Info | KCTD6 | |
| **159** | 0.004191 | 19.2 | 52.1 | 42.3 | 206569 at | interleukin 24 | Info | IL24 | |
| **160** | 0.0041965 | 74.6 | 43.7 | 77.8 | 225338 at | zinc finger, CCHC domain containing 9 | Info | ZCCHC9 | |
| **161** | 0.0042477 | 12.4 | 10 | 26.3 | 203650 at | protein C receptor, endothelial (EPCR) | Info | PROCR | |
| **162** | 0.0042591 | 103.6 | 54 | 88.2 | 222476 at | KIAA1194 | Info | KIAA1194 | |
| **163** | 0.0042876 | 200 | 162.9 | 311.1 | 221602 s at | regulator of Fas-induced apoptosis | Info | TOSO | |
| **164** | 0.0043282 | 74 | 36.8 | 67.6 | 212214 at | optic atrophy 1 (autosomal dominant) | Info | OPA1 | immunology |
| **165** | 0.0043616 | 40.4 | 12.5 | 58.8 | 235400 at | Fc receptor homolog expressed in B cells | Info | FRFB | |
| **166** | 0.0043619 | 98.2 | 39.5 | 91 | 211256 x at | butyrophilin, subfamily 2, member A1 | Info | BTN2A1 | |
| **167** | 0.0044282 | 187.6 | 465.9 | 238 | AFFX-r2-Ec- bioB-5 at | | Info | | |
| **168** | 0.0044636 | 358.6 | 72.4 | 317.3 | 201386 s at | DEAH (Asp-Glu-Ala-His) box polypeptide 15 | Info | DHX15 | |
| **169** | 0.0045185 | 81.4 | 41.7 | 82.4 | 204168 at | microsomal glutathione S- transferase 2 | Info | MGST2 | pharmacology |
| **170** | 0.0045364 | 156 | 305 | 246.3 | 213041 s at | ATP synthase, H+ transporting, mitochondrial F1 complex, delta subunit | Info | ATP5D | |
| **171** | 0.0045462 | 37.8 | 76.9 | 35.9 | 222041 at | DPH2-like 1 (S. cerevisiae) /// candidate tumor suppressor in ovarian cancer 2 | Info | DPH2L1 /// OVCA2 | |
| **172** | 0.0045494 | 58.6 | 33.3 | 30.3 | 204109 s at | nuclear transcription factor E, alpha | Info | NFYA | gene_regulatio n, immunology, transcription |
| **173** | 0.0046367 | 27.3 | 10 | 39.9 | 209602 at | GATA binding protein 3 | Info | GATA3 | gene_regulatio immunology, misc, transcription |
| **174** | 0.0046481 | 214.3 | 225.4 | 129.1 | 228768 at | KIAA1961 protein | Info | KIAA1961 | |
| **175** | 0.0046552 | 21.2 | 25.2 | 37 | 231843 at | DEAD (Asp- Glu-Ala-Asp) box polypeptide | Info | DDX55 | |
| **176** | 0.0047735 | 32.6 | 118.6 | 74.7 | 217390 x at | | Info | | |
| **177** | 0.0047736 | 17.6 | 10 | 22.2 | 240557 at | CDNA FLJ41867 fis, clone OCBBF2005546 | Info | | |
| **178** | 0.0048014 | 57.9 | 101.3 | 65.3 | 217499 x at | | Info | | |
| **179** | 0.0048023 | 300.9 | 170.1 | 280.1 | 220742 s at | N-glycanase 1 | Info | NGLY1 | |
| **180** | 0.00482 | 76.8 | 36.1 | 663.5 | 207629 s at | rho/rac guanine nucleotides exchange factor (GEF) 2 | Info | ARHGEF2 | |
| **181** | 0.0048333 | 10.8 | 14.2 | 29.2 | 238057 at | | Info | | |
| **182** | 0.0048512 | 77.9 | 28.4 | 33.3 | 206618 at | interleukin 18 receptor 1 | Info | IL18R1 | immunology |
| **183** | 0.0048879 | 28.3 | 71.6 | 37.2 | 203389 at | kinesin family member 3C | Info | KIF3C | |
| **184** | 0.0048938 | 41.1 94 | 94 | 67.4 | 243216 x at | | Info | | |
| **185** | 0.0049691 | 45.8 | 70.1 | 36.6 | 208022 s at | CDC14 cell division cycle 14 homolog B (S. cerevisiae) /// CDC14 cell division cycle 14 homolog B (S. cerevisiae) | Info | CDC14B | |

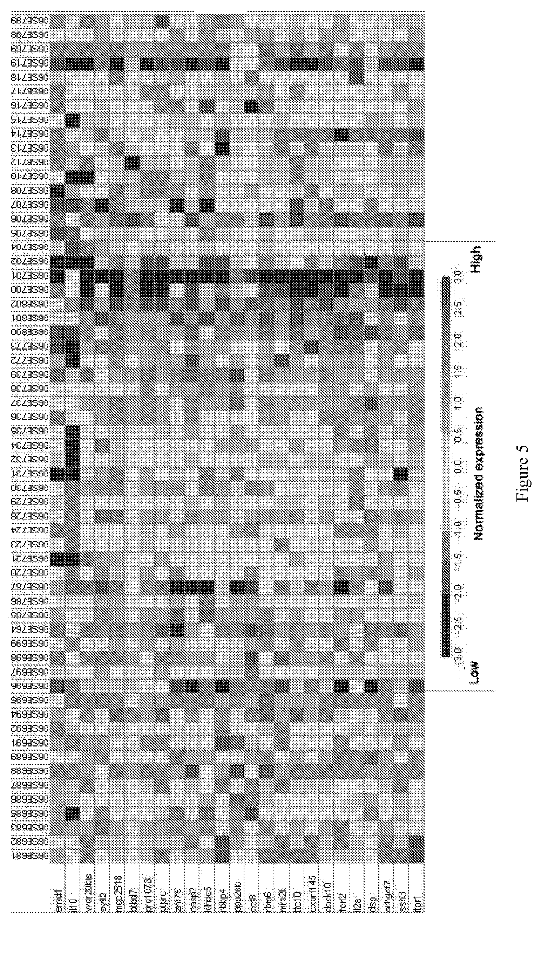

The results of the cluster analysis both for samples and for genes obtained after these class comparisons are shown in Figure 5.

### 2. Validation by real-time PCR and construction of the classifier

Table 10 includes the list of the 95+1 genes and assays selected to configure the LDAs from the results obtained in the screening with DNA chip.

**Table 10: List of genes and assays selected to configure LDA**

| **Assay code** | **Gene symbol** | **Gene name** |
|---|---|---|
| Hs00154040_m1 | ANX4J | annexin A4 |
| Hs0015242_m1 | CASP2 | caspse 2, apoptosis-related cysteine peptidase (neural precursor cell expressed, developmentaly down-regulated 2) |
| Hs00164982_m1 | JAG1 | jagged 1 (Alagille syndrome) |
| Hs0016565_m1 | ATXN1 | ataxin 1 |
| Hs00166163_m1 | CYBB | cytochrome b-245, beta polypeptide (chronic granulomatous disease) |
| Hs00168405_m1 | IL2A | interleukin 12A (natural killer cell stimulatory factor 1, cytoxic lymphocyte maturation factor 1, p35) |
| Hs00168433_m1 | ITGA4 | integrin, alpha 4 (antigen CD4D, alpha 4 subunit of VLA-4 receptor) |
| Hs00168469_m1 | ITGB7 | integrin, beta 7 |
| Hs00169880_m1 | MTM1 | myotubularin 1 |
| Hs00171041_m1 | CXR3 | chemokine (C-X-C motif) receptor 3 |
| Hs00171257_m1 | TGB1 | transforming growth factor, beta 1 (Camurati-Eingelmann disease) |
| Hs00172915_m1 | RBM6 | RNA binding motif protein 6 |
| Hs00173149_m1 | ZNF24 | zinc finger protein 24 (KOX 17) |
| Hs0017319_m1 | ZNF146 | zinc finger protein 146 |
| Hs00173947_m1 | GP1BA | glycoprotein lb (platelet), alpha polypeptide |
| Hs00174086_m1 | IL10 | interleukin 10 |
| Hs00174122_m1 | IL4 | interleukin 4 |
| Hs00174128_m1 | TNF | tumor necrosis factor (TNF superfamily, member 2) |
| Hs00174143_m1 | IFNG | interferon, gamma |
| Hs00174796_m1 | CD28 | CD28 molecule |
| Hs00175480_m1 | CTLA4 | cytotoxic T-lymphocyte-associated protein 4 |
| Hs00175738_m1 | KMO | kynurenine 3-monoxygenase (kynurenine 3-hydroxylase) |
| Hs00177323_m1 | NEK4 | NIMA (never in mitosis gene a)-related kinase 4 |
| Hs00179887_m1 | MSH2 | mutS homolog 2, colon cancer, nonpolyposis type 1 (E. coli) |
| Hs00181881_m1 | ITPR1 | inositol 1,4,5-triphosphate receptor, type 1 |
| Hs00182073_m1 | MX1 | myxovirus (influenza virus) resistance 1, interferon-inducible protein p78 (mouse) |
| Hs00183973_m1 | KIFAP3 | kinesin-associated protein 3 |
| Hs00189422_m1 | DSP | desmoplakin |
| Hs00194836_m1 | TSPAN2 | tetraspanin 2 |
| Hs00197926_m1 | TTC10 | tetratricopeptide repeat domain 10 |
| Hs00203436_m1 | TBX21 | T-box 21 |
| Hs00208425_m1 | HELZ | helicase with zinc finger |
| Hs00211612_m1 | LOC51136 | PTD016 protein |
| Hs00214273_m1 | GIPC2 | GIPC PDZ domain containing family, member 2 |
| Hs00215231_m1 | MRPL16 | mitochondrial ribosomal protein L16 |
| Hs00216842_m1 | BTBD7 | BTB (POZ) domain containing 7 |
| Hs00219525_m1 | DMAP1 | DNA methyltransferase 1 associated protein 1 |
| Hs00219575_m1 | HLA-DRA | major histocompatibility complex, class II, DR alpha |
| Hs00221246_m1 | PRX | periaxin |
| Hs00222575_m1 | FLJ12716 | FLJ12716 protein |
| Hs00223326_m1 | ELSPBP1 | epididymal sperm binding protein 1 |
| Hs00227238_m1 | CXorf45 | chromosome X open reading frame 45 |
| Hs00229156_m1 | FCRL2 | Fc receptor-like 2 |
| Hs00231122_m1 | GATA3 | GATA brinding protein 3 |

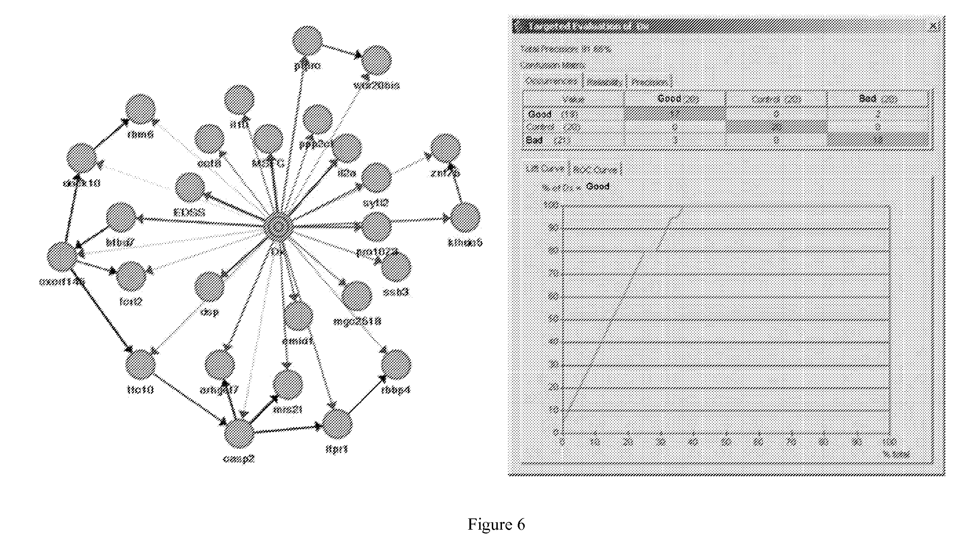

The statistical analysis identified 25 genes which presented significant differences (p<0.01) in the expression levels between the three classes (Figure 6).

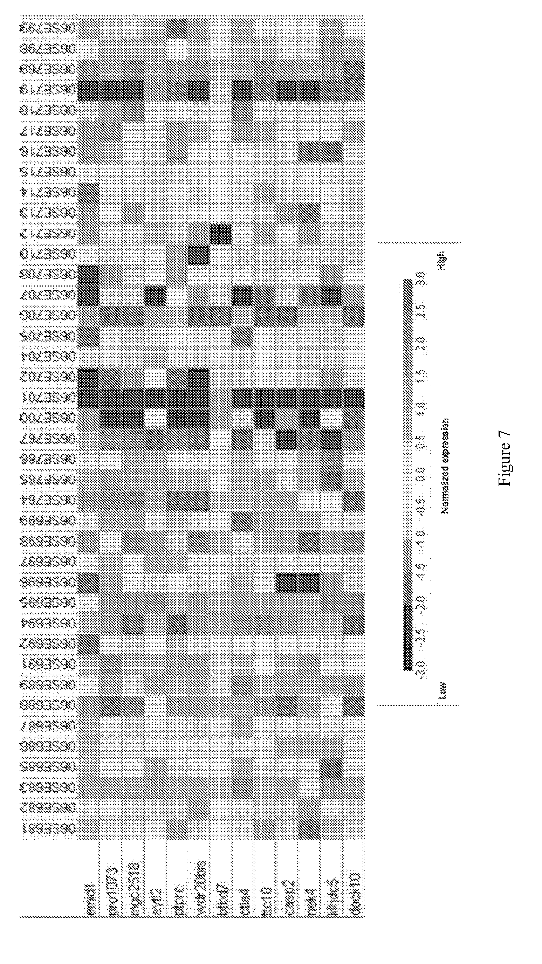

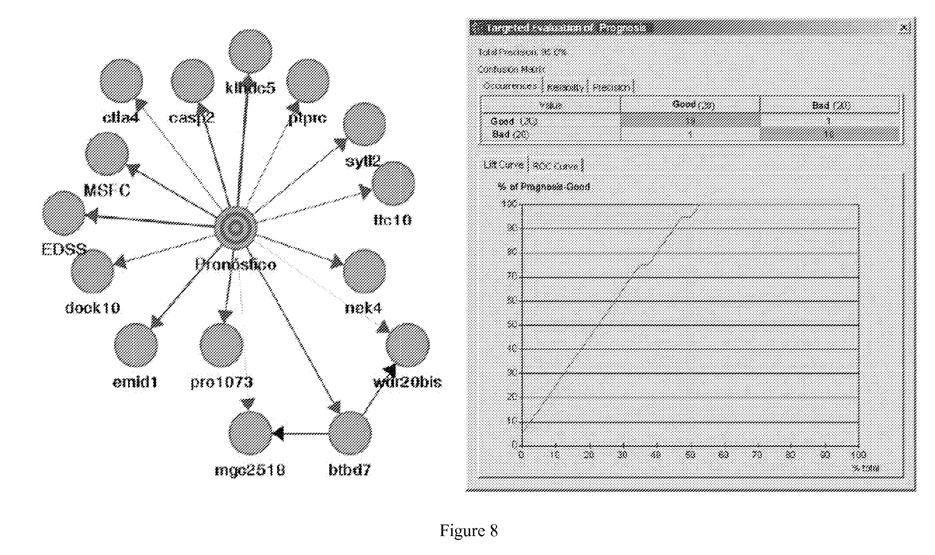

Using these 25 genes and the EDSS and MSFC clinical variables at the onset of the disease, a Bayesian classifier was constructed which showed a precision of 91.66% between the three diagnoses (Figures 7 and 8). This classifier had a precision of 87.5% upon distinguishing between good and bad prognosis.

For the purpose of increasing precision when distinguishing between good and bad prognosis, a new classifier was constructed using the clinical variables and only those genes which presented significant differences (p<0.05) in the expression levels of both classes (Figures 9, 10 and 11). 13 genes were used (Table 11) and the precision that was then obtained was 95%.

**Table 11: Genes differentiating between good and bad prognosis.**

| Assay code | Gene symbol | Gene name |
|---|---|---|
| Hs00216842_m1 | BTBD7 | BTB (POZ) domain containing 7 |
| Hs00154242_ m1C | ASP2 | caspase 2, apoptosis-related cysteine peptidase (neural precursor cell expressed, developmentally down-regulated 2) |
| Hs00175480_m1 | CTLA4 | cytotoxic T-lymphocyte-associated protein 4 |
| Hs00391515_m1 | DOCK10 | dedicator of cytokinesis 10 |
| Hs00294940_m1 | EMID1 | EMI domain containing 1 |
| Hs00325227_m1 | KLHDC5 | kelch domain containing 5 |
| Hs00292260_m1 | MGC25181 | hypothetical protein MGC25181 |
| Hs00177323_m1 | NEK4 | NIMA (never in mitosis gene a)-related kinase 4 |
| Hs00273907_s1 | PR01073,MALAT1 PRO1073 | protein metastasis associated lung adenocarcinoma transcript 1 (non-coding RNA) |
| Hs00365634_g1 | PTPRC | protein tyrosine phosphatase, receptor type, C |
| Hs00262988_m1 | SYTL2 | synaptotagmin-like 2 |
| Hs00197926_m1 | TTC10 | tetratricopeptide repeat domain 10 |
| Hs00375921_m1 | WDR20bis | WD repeat domain 20 |

Table 12 shows the analysis of the information provided by each variable for establishing the prognosis. The weight represents the relative amount of information provided by each variable to the classification of the prognosis. The list of variables is arranged in descending order according to the information provided by each one. The *a priori* modal value describes the most probable value of each variable when the prognosis is unknown, whereas the modal value for each prognosis describes the most probable value for that prognosis. All the modal values are accompanied by their probability. The variation for each prognosis is a measure indicating the difference of probability between the *a priori* modal value and the modal value for the prognosis when the latter is known. The formula used for calculating it is: -log₂ (P (modal value for the prognosis)) + log₂ (P (modal value for the prognosis value observed)). The simple underlined values simple indicate positive variations (the probability of the modal value for the prognosis is greater than that of the *a priori* modal value) whereas the values in *italics* indicate negative variations. Obviously no variation is indicated if the modal value for the prognosis is different from the *a priori* modal value. The modal value for the prognosis is then represented in bold print.

**Table 12: Weight, a priori, bad prognosis and good prognosis modal values for the 13 markers selected and for the EDSS and MSFC clinical variables.**

| Variable | Weight | *A priori* modal value | | Bad prognosis modal value | | Good prognosis modal value | | Bad prognosis variation | Good prognosis variation |
|---|---|---|---|---|---|---|---|---|---|
| klhdc5 | 1.0000 | <=-2.640 | 42.50% | <=-**3.145** | 60.00% | <=-2.640 | 65.00% | | 0.6130 |
| EDSS | 0.8512 | <=2.000 | 50.00% | >**2.000** | 85.00% | <=2.000 | 85.00% | | 0.7655 |
| casp2 | 0.6791 | <=-2.385 | 67.50% | <=-2.385 | 90.00% | <=**-1.920** | 50.00% | 0.4150 | |
| emidl | 0.6367 | >-3.295 | 62.63% | <=**-3.295** | 66.67% | >-3.295 | 91.92% | | 0.5536 |
| MSFC | 0.5951 | <=0.665 | 77.50% | <=0.665 | 100.00% | <=0.665 | 55.00% | 0.3677 | *-0.4948* |
| pro1073 | 0.5951 | >-2.765 | 77.50% | >-2.765 | 55.00% | >-2.765 | 100.00% | *-0.4948* | 0.3677 |
| btbd7 | 0.5273 | >-2.825 | 70.00% | <=**-2.825** | 55.00% | >-2.825 | 95.00% | | 0.4406 |
| mgc2518 | 0.4406 | >-3.585 | 82.50% | >-3.585 | 65.00% | >-3.585 | 100.00% | *-0.3440* | 0.2775 |
| wdr20bis | 0.4406 | >-2.740 | 82.50% | >-2.740 | 65.00% | >-2.740 | 100.00% | *-0.3440* | 0.2775 |
| nek4 | 0.3978 | <=-2.335 | 54.47% | <=-2.335 | 78.95% | >**-2.335** | 70.00% | 0.5353 | |
| syt12 | 0.3902 | <=-2.650 | 67.50% | <=-2.650 | 90.00% | >**-2.650** | 55.00% | 0.4150 | |
| dock10 | 0.3691 | >-3.320 | 85.00% | >-3.320 | 70.00% | >-3.320 | 100.00% | *-0.2801* | 0.2345 |
| ttc10 | 0.3066 | >-2.735 | 77.50% | >-2.735 | 60.00% | >-2.735 | 95.00% | *-0.3692* | 0.2937 |
| ptprc | 0.3009 | >-3.210 | 87.50% | >-3.210 | 75.00% | >-3.210 | 100.00% | *-0.2224* | 0.1926 |
| ctla4 | 0.2860 | >-3.720 | 59.44% | <=**-3.720** | 61.11% | >-3.720 | 80.00% | | 0.4285 |

Table 13 presents the precision of the classifier as the number of variables making it up increases. The variables are introduced in order according to the information provided by each one for establishing the prognosis. Within the prognoses the values are presented as correctly classified individuals (correct) with respect to the incorrectly classified individuals (incorrect).

**Table 13: Precision of the classifier as genes and clinical variables are incorporated.**

| Variable | Precision | Good prognosis (correct / incorrect) | Bad prognosis (correct / incorrect) |
|---|---|---|---|
| klhdc5 | 85.00% | 18 / 2 | 16 / 4 |
| + | | | |
| EDSS | 90.00% | 16 / 4 | 20 / 0 |
| + | | | |
| casp2 | 92.50% | 17 / 3 | 20 / 0 |
| + | | | |
| emidl | 92.50% | 17 / 3 | 20 / 0 |
| + | | | |
| MSFC | 90.00% | 16 / 4 | 20 / 0 |
| + | | | |
| pro1073 | 95.00% | 18 / 2 | 20 / 0 |
| + | | | |
| btbd7 | 95.00% | 19 / 1 | 19 / 1 |
| + | | | |
| mgc2518 | 92.50% | 18 / 2 | 19 / 1 |
| + | | | |
| wdr20bis | 95.00% | 19 / 1 | 19 / 1 |
| + | | | |
| nek4 | 92.50% | 18 / 2 | 19 / 1 |
| + | | | |
| syt12 | 92.50% | 18 / 2 | 19 / 1 |
| + | | | |
| dock10 | 92.50% | 18 / 2 | 19 / 1 |
| + | | | |
| ttc10 | 95.00% | 19 / 1 | 19 / 1 |
| + | | | |
| ptprc | 92.50% | 18 / 2 | 19 / 1 |
| + | | | |
| ctla4 | 95.00% | 19 / 1 | 19 / 1 |

Table 14 presents the conditional probabilities for the prognosis of the disease for each variable used by the classifier.

**Table 14: conditional probabilities for the prognosis of the disease for each variable used by the classifier**

| Variable | Modal value | *A priori* probability | Probability of a bad prognosis | Probability of a good prognosis |
|---|---|---|---|---|
| | <=-3.145 | 35.14% | 60.00% | 10.00% |
| klhdc5 | <=-2.640 | 42.37% | 20.00% | 65.00% |
| | <=-2.475 | 10.06% | 20.00% | 0.00% |
| | >-2.475 | 12.43% | 0.00% | 25.00% |
| | <=2.000 | 49.80% | 15.00% | 85.00% |
| EDSS | >2.000 | 50.20% | 85.00% | 15.00% |
| | <=-2.385 | 67.63% | 90.00% | 45.00% |
| casp2 | <=-1.920 | 24.86% | 0.00% | 50.00% |
| | >-1.920 | 7.51% | 10.00% | 5.00% |
| | <=-3.295 | 37.54% | 66.67% | 8.08% |
| emidl | >-3.295 | 62.46% | 33.33% | 91.92% |
| MSFC | <=0.665 | 77.63% | 100.00% | 55.00% |
| | >0.665 | 22.37% | 0.00% | 45.00% |
| | <=-2.765 | 22.63% | 45.00% | 0.00% |
| pro1073 | >-2.765 | 77.37% | 55.00% | 100.00% |
| btbd7 | <=-2.825 | 30.14% | 55.00% | 5.00% |
| | >-2.825 | 69.86% | 45.00% | 95.00% |
| mgc2518 | <=-3.585 | 17.60% | 35.00% | 0.00% |
| | >-3.585 | 82.40% | 65.00% | 100.00% |
| | <=-2.740 | 17.60% | 35.00% | 0.00% |
| wdr20bis | >-2.740 | 82.40% | 65.00% | 100.00% |
| | <=-2.335 | 54.61% | 78.95% | 30.00% |
| nek4 | >-2.335 | 45.39% | 21.05% | 70.00% |
| | <=-2.650 | 67.63% | 90.00% | 45.00% |
| syt12 | >-2.650 | 32.37% | 10.00% | 55.00% |
| dock10 | <=-3.320 | 15.09% | 30.00% | 0.00% |
| | >-3.320 | 84.91% | 70.00% | 100.00% |
| | <=-2.735 | 22.60% | 40.00% | 5.00% |
| ttc10 | >-2.735 | 77.40% | 60.00% | 95.00% |
| ptprc | <=-3.210 | 12.57% | 25.00% | 0.00% |
| | >-3.210 | 87.43% | 75.00% | 100.00% |
| ctla4 | <=-3.720 | 41.22% | 61.11% | 20.00% |
| | >-3.720 | 58.78% | 38.89% | 80.00% |

## Claims

1. An *in vitro* method for determining the clinical prognosis of a patient who has multiple sclerosis which comprises
(a) comparing
(i) the value corresponding to the expression of a gene selected from the group of KLHDC5, CASP2, EMID1, PRO1073, BTBD7, MGC2518, WDR20bis, NEK4, SYLT2, DOCK10, TTC10, PTPRC and CTLA4 with a table of conditional probabilities between ranges of modal values of the expression of said genes and probability values that the multiple sclerosis has a good or bad prognosis and/or
(ii) the value of a clinical variable selected from the group of EDSS and MSFC with a table of conditional probabilities between ranges of modal values of said clinical variables and probability values that the multiple sclerosis has a good or bad prognosis and
(b) assigning a probability of a bad and a good prognosis corresponding to the probability associated with the range in which the value of the expression or of the clinical variable is located.

2. An *in vitro* method for determining the clinical prognosis of a patient who has sclerosis which comprises
(a) comparing
(i) the values corresponding to the expression of at least two genes selected from the group of KLHDC5, CASP2, EMID1, PRO1073, BTBD7, MGC2518, WDR20bis, NEK4, SYLT2. DOCK10, TTC10, PTPRC and CTLA4 with a table of conditional probabilities between ranges of modal values of the expression of said genes and probability values that the multiple sclerosis has a good or bad prognosis and/or
(ii) the values of the EDSS and MSFC clinical variables with a table of conditional probabilities between ranges of modal values of said clinical variables and probability values that the multiple sclerosis has a good or bad prognosis and
(b) assigning a probability of a bad prognosis corresponding to the conditional probability of a bad prognosis associated with the ranges of modal values in which the expression values of each of the genes the expression of which has been determined and/or the clinical variables determined are located, and assigning a probability of a good prognosis corresponding to the conditional probability of a good prognosis associated with the ranges of modal values in which the expression values for each of the genes the expression of which has been determined and/or the clinical variables determined are located.

3. A method according to claim 2, wherein the expression values of at least 3, at least 4, at least 5, at least 6, at least 7, at least 8, at least 9, at least 10 and at least 11 genes are determined.

4. A method according to claim 2 or 3, wherein the expression values of the KLHDC5 gene and of the EDSS clinical variable are determined.

5. A method according to claim 4, wherein the expression value of the CASP2 gene is additionally determined.

6. A method according to claim 5, wherein the expression value of the EMID1 gene is additionally determined.

7. A method according to claim 6, wherein the value of the MSFC clinical variable is additionally determined.

8. A method according to claim 7, wherein the expression value of the PRO1073 gene is additionally determined.

9. A method according to claim 8, wherein the expression value of the BTBD7 gene is additionally determined.

10. A method according to claim 9, wherein the expression value of the MGC2518 gene is additionally determined.

11. A method according to claim 10, wherein the expression value of the WDR20bis gene is additionally determined.

12. A method according to claim 11, wherein is the expression value of the NEK4 gene additionally determined.

13. A method according to claim 12, wherein the expression value of the SYLT2 gene is additionally determined.

14. A method according to claim 13, wherein the expression value of the DOCK10 gene is additionally determined.

15. A method according to claim 14, wherein the expression value of the TTC10 gene is additionally determined.

16. A method according to claim 15, wherein the expression value of the PTPRC gene is additionally determined.

17. A method according to claim 16, wherein the expression value of the CTLA4 gene is additionally determined.

18. The method according to any of claims 1 to 17, wherein the determination of the expression values of the different genes is determined by means of real-time PCR.

19. The method according to any of claims 1 to 17, wherein the table of conditional probabilities between the expression levels of each of the genes and the probability values that the multiple sclerosis has a good or bad prognosis and between the modal values of each of the clinical variables and the probability values that the multiple sclerosis has a good or bad prognosis are those indicated in Table 14.

20. A method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from the group of genes listed in positions 3, 5, 6, 7, 9, 11, 13, 16, 19, 20, 22, 24, 25, 26, 30, 31, 33, 34, 35, 37, 41 or 43 of Table 3, or of the polypeptides encoded by said genes, in a biological sample isolated from the patient and
(b) comparing the expression levels of said genes or of said polypeptides with a reference value calculated from one or several samples obtained from a healthy patient
wherein
(i) an increase of the expression of the genes in position 6, 7, 9, 33, 35, 37 or 43, or of the polypeptides encoded by said genes, or a reduction of the expression of the genes in position 3, 5, 11, 13, 16, 19, 22, 24, 25, 26, 30, 31, 34, 41, or of the polypeptides encoded by said genes with respect to the reference value, is indicative of a bad prognosis of multiple sclerosis in said subject, that the therapy is ineffective or that the patient is selected for an aggressive therapy or
(ii) an increase of the expression of the genes in positions 3, 5, 11, 16, 20, 30, or of the polypeptides encoded by said genes, or a reduction of the expression of the gene in position 43, or of the polypeptide encoded by said gene with respect to the reference value, is indicative of a good prognosis of multiple sclerosis in said patient, that the therapy is effective or that the patient is selected to not receive therapy or to receive a rather non-aggressive therapy.

21. A method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from the group of genes listed in positions 1 to 21 of Table 5, or of the polypeptides encoded by said genes, in a biological sample isolated from the patient and
(b) comparing the expression levels of said genes or of said polypeptides with a reference value calculated from one or several samples obtained from patients with a good prognosis and with a reference value calculated from one or several samples obtained from patients with a bad prognosis
wherein
(i) an increase of the expression of the genes in position 1, 2, 3, 4, 5, 8, 9, 10, 14, 19, 20 or 21 or of the polypeptides encoded by said genes with respect to a reference value obtained from one or several samples from patients diagnosed with multiple sclerosis with a bad prognosis is indicative of a good prognosis of multiple sclerosis in said subject, that the therapy is effective or that the patient is selected to not receive an aggressive therapy and
(ii) an increase of the expression of the genes in positions 6, 7, 11, 12, 13, 15, 16, 17 or 18 or of the polypeptides encoded by said genes with respect to a reference value obtained from one or several samples from patients diagnosed with multiple sclerosis with a good prognosis is indicative of a bad prognosis of multiple sclerosis in said patient, that the therapy is not effective or that the patient is selected to receive therapy or to receive a rather non-aggressive therapy.

22. A method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from Table 6, or of the polypeptides encoded by said genes, in a sample isolated from the patient and
(b) comparing the expression levels of said genes with a reference value
calculated from one or several samples obtained from a healthy patient wherein an increase of the expression of the genes in position 4, 8, 11, 13, 15, 18, 19, 20, 21, 24, 25, 28, 30 or 32, or of the polypeptides encoded by said genes, or a reduction of the genes in position 1, 2, 3, 5, 6, 7, 9, 10, 12, 14, 16, 17, 22, 23, 26, 27, 29 or 31, or of the polypeptides encoded by said genes, with respect to the reference value is indicative of a bad prognosis of multiple sclerosis, that the therapy is not effective or that the patient is selected for an aggressive therapy.

23. A method for determining the clinical prognosis of a subject who has multiple sclerosis, for monitoring the effect of the therapy administered to a subject who has multiple sclerosis or for assigning a customized therapy to a subject who has sclerosis which comprises
(a) determining the expression level of one or several genes selected from Table 7, or of the polypeptides encoded by said genes, in a sample isolated from the patient
(b) comparing the expression levels of said genes with a reference value calculated from one or several samples obtained from a healthy patient
wherein an increase of the expression of the genes in position 2, 5, 6, 7, 8 and 10, or of the polypeptides encoded by said genes, or a reduction of the expression of the genes in position 1, 3, 4 or 9, or of the polypeptides encoded by said genes, with respect to the reference value is indicative of a good prognosis of multiple sclerosis or that the therapy administered is effective or that the patient is selected to not receive therapy or to receive a rather non-aggressive therapy.

24. A method for diagnosing multiple sclerosis in a subject which comprises
(a) determining the expression level of one or several genes selected from the group of genes indicated in Table 8, or of the polypeptides encoded by said genes, in a sample isolated from the subject
(b) comparing the expression levels of said genes with a reference value calculated from one or several samples obtained from a healthy patient
wherein a reduction of the expression of the genes in position 1, 2, 6, 10, 15 or 16, or of the polypeptides encoded by said genes, or an increase in the expression of the genes in position 3, 4, 5, 7, 8, 9, 11, 12, 13 or 14, or of the polypeptides encoded by said genes, with respect to the reference value is indicative that the subject suffers multiple sclerosis.

25. A method according to any of claims 19 to 24, wherein the reference value is obtained from a tissue sample obtained from a healthy subject.

26. A method according to any of claims 19 to 25, wherein the sample or samples comes or come from a patient who has suffered a single flare-up of multiple sclerosis, from a patient suffering RR-MS, from a patient suffering PP-MS, from a patient suffering SP-MS and of a patient of PR-MS.

27. A method according to any of claims 19 to 26, wherein the determination of the expression levels of the genes is carried out in a blood sample.

28. A method according to claim 27, wherein the determination of the expression levels is carried out in a preparation of peripheral blood mononuclear cells.

29. A method according to any of claims 19 to 28, wherein the determination of the expression levels is carried out by means of real-time PCR.

30. A kit comprising a set of probes, wherein said set comprises a probe specific for each of the genes indicated in at least one table selected from the group of Tables 3, 5-8 and 11.

31. A kit according to claim 30, wherein the set of probes consists of a probe specific for each of the genes indicated in at least one table selected from the group of Tables 3, 5-8 and 11.

32. A kit according to claim 31, wherein the set of probes consists of a probe specific for each of the genes indicated in Table 11.

33. A kit according to any of claims 30 to 32, wherein the kit additionally comprises at least one probe specific for a reference gene with constitutive expression.

34. A kit according to claim 33, wherein the at least one reference gene is selected from the group of GABPA, UBC, beta-actin and beta-microglobulin.

35. A kit according to any of claims 30 to 34, wherein the probes form part of an array.

36. A kit according to claim 35, wherein the array is an LDA (low-density array).

37. Use of a kit according to any of claims 30 to 36 for determining the prognosis of a patient diagnosed with multiple sclerosis, for determining the effectiveness of a treatment for multiple sclerosis or for diagnosing multiple sclerosis in a patient.
